(19) Europäisches Patentamt
European Patent Office
Office européen des brevets

(11) EP 4 613 098 A2

(12) EUROPEAN PATENT APPLICATION

(43) Date of publication:
**10.09.2025 Bulletin 2025/37**

(21) Application number: **25191126.9**

(22) Date of filing: **12.09.2021**

(51) International Patent Classification (IPC):
***A01N 43/90*** *(2006.01)*

(52) Cooperative Patent Classification (CPC):
**C05G 3/90; A01N 43/76; A01N 43/78; A01N 43/90; C07D 263/16; C07D 263/46; C07D 277/16; C07D 277/36; C07D 277/56; C07D 417/12; C07D 417/14; C07D 471/04; C07D 513/04;**
Y02P 60/21

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.09.2020 EP 20196033**
**05.02.2021 EP 21155568**

(62) Document number(s) of the earlier application(s) in accordance with Art. 76 EPC:
**21805375.9 / 4 211 097**

(27) Previously filed application:
**12.09.2021 EP PCT/EP2021/075025**

(71) Applicant: **EuroChem Antwerpen**
**2040 Antwerpen (BE)**

(72) Inventors:
- **HABERMÜLLER, Heiko**
  **69469 Weinheim (DE)**
- **HENKE, Catarina**
  **68163 Mannheim (DE)**
- **Thomas, Mannheim**
  **68165 Mannheim (DE)**
- **PETERS, Nils**
  **68165 Mannheim (DE)**
- **BEECKMAN, Fabian**
  **9052 Gent (BE)**
- **MOTTE, Hans**
  **9052 Gent (BE)**
- **DROZDZECKI, Andrzej**
  **9052 Gent (BE)**
- **AUDENAERT, Dominique**
  **9052 Gent (BE)**
- **BEECKMAN, Tom**
  **9052 Gent (BE)**

(74) Representative: **Willnegger, Eva**
**Montgomery IP**
**Boulevard Saint Michel 10/4**
**1150 Bruxelles (BE)**

Remarks:
This application was filed on 22-07-2025 as a divisional application to the application mentioned under INID code 62.

# (54) N-HETEROCYCLIC COMPOUNDS USED AS NITRIFICATION INHIBITOR

(57) The use of an N-heterocyclic compound of the general formula (a)

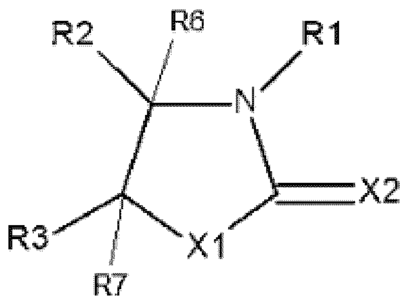

(a)

as nitrification inhibitor.

EP 4 613 098 A2

## Description

The present invention relates to the use of a specific N-heterocyclic compound as nitrification inhibitor or as nitrogen stabilizer, to fertilizer mixtures containing it, a process for its preparation, and the method of fertilizing soil including its application.

In order to provide plants in agriculture or horticulture with the nitrogen they need, fertilizers comprising ammonium compounds are frequently used.

With the invention of the Haber-Bosch process in 1908, fertilizers pushed the Green Revolution in the 1950s and 60s and helped to feed the exponentially growing world population. As nitrogen (N) is one of the most important nutrients for plants to grow, this industrial synthesis of ammonia ($NH_3$) increased the limits of the earth to produce food crops. However, the N-sources, mainly ammonium ($NH_4^+$) and nitrate ($NO_3^-$), that were used for fertilization, were not only used by plants, but also leached into the environment or were microbially converted via the nitrogen cycle into inaccessible N forms. As such, there is an average N fertilizer loss of almost 50%. Moreover, N-leaching leads to eutrophication of groundwater and free water and causes toxic algal blooms, decreased value of recreational water or contamination of drinking water. Microbial conversion into the greenhouse gas $N_2O$ strongly enhances global warming. However, with a global population expected to reach 9.7 billion by 2050, the FAO expects fertilizer demands to increase up to 50% or more. Hence, mitigation strategies are important to limit the amount of N-emissions as much as possible. One approach is to inhibit nitrification, i.e. the conversion of $NH_4^+$ or $NH_3$ into $NO_3^-$.

In contrast to $NO_3^-$, the positively charged $NH_4^+$ binds to the negatively charged soil particles and will hardly leach from the soil. Therefore, $NH_4^+$ is the preferred N-source and should be kept available in soil for plant uptake. $NH_4^+$ may however be converted into $NO_3^-$ via nitrification, a microbial process of the global N cycle whereby $NH_3$, which is in a pH-dependent equilibrium with $NH_4^+$, is converted into $NO_3^-$ via nitrite ($NO_2^-$) by soil micro-organisms. Hence, nitrification is a central step that causes the entering of $NH_4^+$ in the N cycle and its subsequent conversion into undesired leachable or volatile N-forms.

The first step in nitrification, $NH_3$ oxidation, is in agricultural soils and substrates predominantly performed by ammonia-oxidizing bacteria (AOB). They first oxidize $NH_3$ into hydroxylamine ($NH_2OH$), which is catalyzed by the ammonia-monooxygenase (AMO) enzyme. Subsequently, the hydroxylamine oxidoreductase (HAO) enzyme catalyzes the second step: $NH_2OH$ oxidation to form nitrite ($NO_2^-$).

Historically, various organic and inorganic compounds were identified as nitrification inhibitors, but only a few are currently commercially distributed, including dicyandiamide (DCD), nitrapyrin (2-chloro-6-(trichloromethyl)-pyridine) or 3,4-dimethylpyrazole phosphate (DMPP). Effectiveness of nitrification inhibitors can, however, vary between different strains and genera of AOB. Therefore, it is important to expand the range of applied nitrification inhibitors.

One problem attending the use of pyrazole compounds as nitrification inhibitors is their high volatility. When fertilizer preparations containing pyrazole compounds are stored, there is a continuous loss of active ingredient as a result of evaporation. For this reason the pyrazole compounds must be formulated in a nonvolatile form by means of appropriate measures.

EP-B-1 120 388 describes phosphoric acid addition salts of 3,4-dimethylpyrazole and 4-chloro-3-methylpyrazole for use as nitrification inhibitors. Through the salt form it is possible for the volatility to be significantly reduced.

WO 96/24566 relates to the use of low-volatility pyrazole derivatives having hydrophilic groups as nitrification inhibitors. As an example, 2-(N-3-methylpyrazole)succinic acid (DMPSA) is proposed as a nitrification inhibitor. Suitable mineral fertilizers cited are ammonium-containing nitrates, sulfates or phosphates.

WO 2011/032904 and WO 2013/121384 describe pyrazole derivatives as nitrification inhibitors, one of which is DMPSA.

A. Saha et al. describe in J. Heterocyclic. Chem., 47, 838 (2010) the green synthesis of 5-substituted-1-3,4-thiadiazole-2-thiols as new potent nitrification inhibitors. The 1,3,4-thiaidazole-2-thiols are 5-substituted by alkyl or aryl residues. The compounds were screened for their *in vitro* nitrification inhibitory activity. Compounds that are 5-substituted by heptyl, 2-chloro phenyl, 2,4-dichloro phenyl, 2-methyl phenyl, 3-methyl phenyl, 3,4-dimethoxy phenyl, 2-hydroxy phenyl, 4-hydroxy-3-methoxy phenyl are considered to be promising nitrification inhibitors.

WO 2020/020765 A1 discloses the use of substituted thiazolidine compounds as nitrification inhibitor. The compounds are derived from 1,3-thiozolidine-2-thione as shown in the table on page 71.

WO 2020/020777 A1 discloses substituted 2-thiazoline as nitrification inhibitors. The compounds are derived from 2-mercapto-2-thiazoline by substituting hydrogen atoms of the ring structure or the mercapto group.

CN 103 524 159 A discloses that among other ingredients oxazolidinthione can be present in the fertilizer. No function for this compound is mentioned.

There is a continued need for new nitrification inhibitors which have a high nitrification inhibitory activity, low toxicity and low volatility.

The object underlying the present invention is therefore to provide novel nitrification inhibitors which preferably have a high nitrification inhibitory activity.

A further object of the present invention is to provide a fertilizer mixture containing such nitrification inhibitor, a process for its preparation, and a method of fertilizing soils employing it.

The objects are achieved by the use of an N-heterocyclic compound of the general formula (a) or (b)

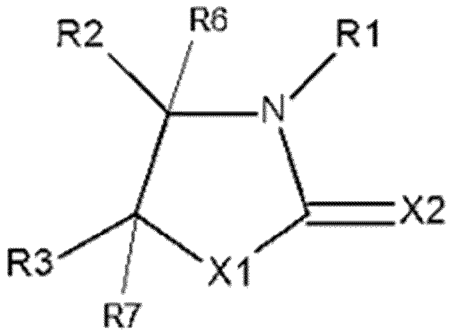

(a)

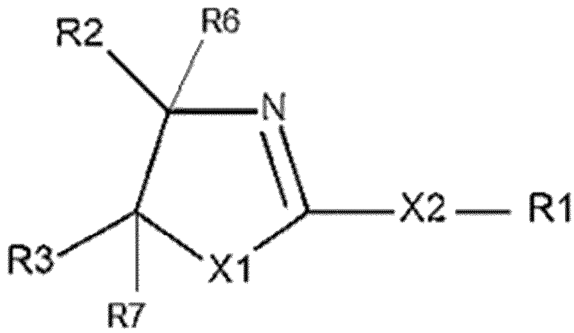

(b)

with the following definitions:

$X^1$ being S or O, $X^2$ being S or O and at least one of $X^1$ and $X^2$ being S

$R^2$ H or $C_{1-4}$-alkyl,

$R^3$ H or $C_{1-4}$-alkyl

$R^6$ and $R^7$ are hydrogen or together form a covalent carbon-carbon bond

in general formula (a) $R^1$ being H, $C_{1-12}$-alkyl or $-CH_2-NR^4R^5$ with

$R^4$ hydrogen or $C_{1-4}$-alkyl,

$R^5$ $C_{1-12}$-hydrocarbon residue which can contain one to three halogen atoms and/or one to four heteroatoms, selected from the group consisting of nitrogen, oxygen and sulfur, it also being possible for $R^4$ and $R^5$, together with the nitrogen atom joining them, to form a 5- or 6-membered saturated or unsaturated heterocyclic radical, which optionally may also contain one or two further heteroatoms selected from the group consisting of nitrogen, oxygen and sulfur,

in general formula (b) $R^1$ being H or $C_{1-17}$-hydrocarbon, preferably H, or $-CH_2-R^5$ with $R^5$ being H or $C_{1-16}$-hydrocarbon residue, which hydrocarbon can contain one to three halogen atoms and/or one to six heteroatoms, selected from the group consisting of nitrogen, oxygen and sulfur,

and preferably in general formula (a) and (b) $X^1$ and $X^2$ being S,

as nitrification inhibitor.

The objects are preferably achieved by the use of an N-heterocyclic compound of the general formula (I) or (II) or (III) or (IV)

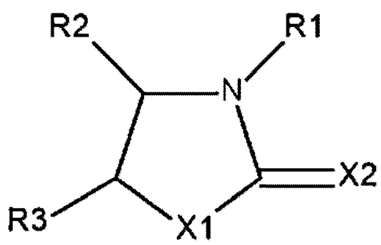

(I)

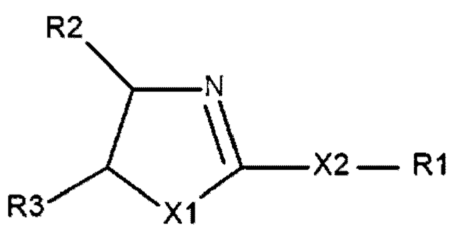

(II)

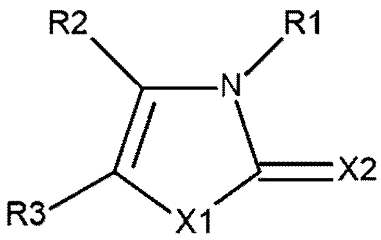

(III)

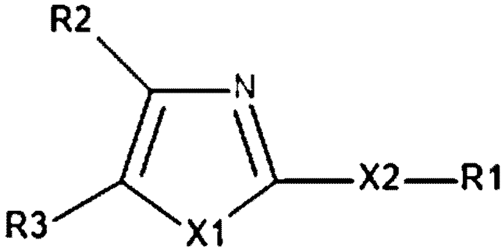

(IV)

with the following definitions:

$X^1$ being S or O, $X^2$ being S or O and at least one of $X^1$ and $X^2$ being S

$R^2$ H or $C_{1-4}$-alkyl,

$R^3$ H or $C_{1-4}$-alkyl

in general formula (I) and (III) $R^1$ being H, $C_{1-12}$-alkyl or $-CH_2-NR^4R^5$ with

$R^4$ hydrogen or $C_{1-4}$-alkyl,

$R^5$ $C_{1-12}$-hydrocarbon residue which can contain one to three halogen atoms and/or one to four heteroatoms, selected from the group consisting of nitrogen, oxygen and sulfur, it also being possible for $R^4$ and $R^5$, together with the nitrogen atom joining them, to form a 5- or 6-membered saturated or unsaturated heterocyclic radical, which optionally may also contain one or two further heteroatoms selected from the group consisting of nitrogen, oxygen and sulfur,

in general formula (II) and (IV) $R^1$ being H or $C_{1-17}$-hydrocarbon, preferably H, or $-CH_2-R^5$ with $R^5$ being H or $C_{1-16}$-hydrocarbon residue, which hydrocarbon can contain one to three halogen atoms and/or one to six heteroatoms, selected from the group consisting of nitrogen, oxygen and sulfur,

and preferably in general formula (II), (III) and (IV) $X^1$ and $X^2$ being S,

as nitrification inhibitor, preferably targeting AOB and possibly comammox.

In the following, reference is often made to N-heterocyclic compounds of the general formulae (I) to (IV). The compounds of the above-mentioned general formulae (a) and (b) can be employed likewise and the references are also valid for formulae (a) and (b). Formula (a) is a combination of formula (I) and (III). Formula (b) is a combination of formula (II) and (IV).

Therein, the N-heterocyclic compound of the general formula (I) and/or (II) and/or (III) and/or (IV) acts as nitrification inhibitor on solid or in liquid fertilizers. They furthermore also act as nitrogen stabilizer in liquid fertilizers or manure. Fertilizers can be organic and/or inorganic and/or organomineral fertilizers.

The object is furthermore achieved by the use of the N-heterocyclic compound of general formula (I) and/or (II) and/or (III) and/or (IV) as an additive or coating material for, preferably inorganic, fertilizers, more preferably ammonium- and/or urea-containing nitrogen fertilizers.

The object is furthermore achieved by the use of the N-heterocyclic compound of general formula (I) and/or II and/or (III) and/or (IV) for reducing the nitrogen or carbon losses in inorganic and/or organic and/or organomineral fertilizers or nitrogen- or carbon-containing compounds or materials and also on harvest refuse and on grazed land or during the storage of liquid manure, and for lowering the ammonia load in animal stalls.

It may furthermore be advantageous to employ the N-heterocyclic compound of general formula (I) and/or (II) and/or (III) and/or (IV) nitrification inhibitor in combination with an additional agrochemical agent, preferably selected from the group consisting of

- at least one further nitrification inhibitor, preferably selected from the group consisting of 2-(3,4-dimethyl-pyrazol-1-yl)-succinic acid (DMPSA), 3,4-dimethylpyrazole (DMP), 3,4-dimethylpyrazolephosphate (DMPP), dicyandiamide (DCD), 1H-1,2,4-triazole, 3-methylpyrazole (3-MP), 2-chloro-6-(trichloromethyl)-pyridine, 5-ethoxy-3-trichloromethyl-1,2,4-thiadiazol, 2-amino-4-chloro-6-methyl-pyrimidine, 2-mercapto-benzothiazole, 2-sulfanilamidothiazole, thiourea, sodium azide, potassium azide, 1-hydroxypyrazole, 2-methylpyrazole-1-carboxamide, 4-amino-1,2,4-triazole, 3-mercapto-1,2,4-triazole, 2,4-diamino-6-trichloromethyl-5-triazine, carbon bisulfide, ammonium thiosulfate, sodium trithiocarbonate, 2,3-dihydro-2,2-dimethyl-7-benzofuranol methyl carbamate and N-(2,6-dimethylphenyl)-N-(methoxyacetyl)-alanine methyl ester, AOA inhibitors or comammox inhibitors,

- at least one urease inhibitor, preferably selected from N-n-butylthiophosphoric triamide (NBTPT) and/or N-n-propylthiophosphoric triamide (NPTPT),

- at least one customary agrochemical auxiliary agent, preferably selected from the group consisting of aqueous and/or organic solvents, pH-adjusting agents, surfactants, wetting agents, spreading agents, adhesion promoters, carriers, fillers, viscosity-adjusting agents, emulsifiers, dispersants, sequestering agents, anti-settling agents, coalescing agents, rheology modifiers, defoaming agents, photo-protectors, anti-freeze agents, biostimulants, pesticides, biocides, plant growth regulators, safeners, penetrants, anticaking agents, mineral and/or vegetable oils and/or waxes, colorants and drift control agents,

and mixtures thereof.

When the additional nitrification inhibitor is employed, the weight ratio of the N-heterocyclic compound of general formula (I) and/or (II) and/or (III) and/or (IV) to the further nitrification inhibitor is preferably 0.1 to 10 : 1, more preferably 0.2 to 5 : 1, most preferably 0.5 to 2 : 1.

Therefore, the nitrification inhibitor according to the present invention can be advantageously used together with or combined with or in admixture with further nitrification inhibitors which are preferably inhibiting ammonia-oxidizing bacteria (AOB) or archaea (AOA), for example ammonia-binding liquid inoculum (ABIL), or complete ammonia oxidation (comammox) bacteria.

Furthermore, specifically if the inorganic fertilizer contains urea, the nitrification inhibitor can also be used together or combined with or in admixture with an urease inhibitor, which is preferably selected from N-n-butylthiophosphoric triamide (NBTPT or NBPT) and/or N-n-propylthiophosphoric triamide (NPTPT or NPPT).

If the N-heterocyclic compound of general formula (I) and/or (II) and/or (III) and/or (IV) of the present invention is combined with N-n-butylthiophosphoric triamide (NBTPT) and/or N-n-propylthiophosphoric triamide (NPTPT), the weight ratio of nitrification inhibitor(s) to urease inhibitor is preferably in the range of from 0.1 to 10 : 1, more preferably 0.5 to 8 : 1, most preferably 1 to 6 : 1.

Therefore, the invention also relates to a mixture, containing at least one N-heterocyclic compound of the general formula (I) or (II) or (III) or (IV)

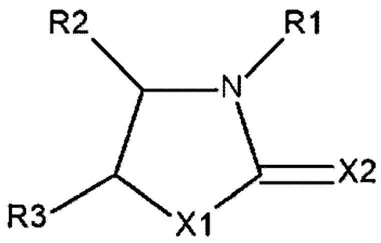
(I)

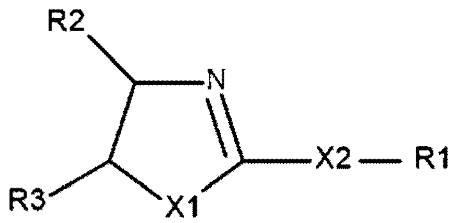
(II)

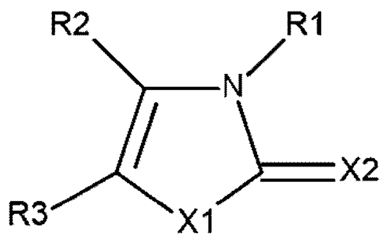
(III)

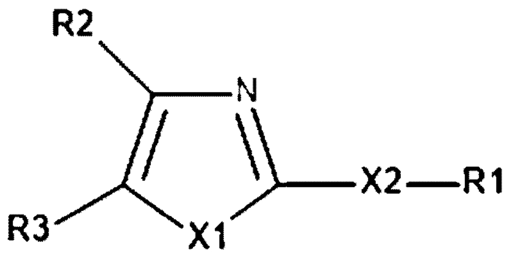
(IV)

with the following definitions:

$X^1$ being S or O, $X^2$ being S or O and at least one of $X^1$ and $X^2$ being S

$R^2$ H or $C_{1-4}$-alkyl,

$R^3$ H or $C_{1-4}$-alkyl

in general formula (I) and (III) $R^1$ being H, $C_{1-12}$-alkyl or $-CH_2-NR^4R^5$ with

$R^4$ hydrogen or $C_{1-4}$-alkyl,

$R^5$ $C_{1-12}$-hydrocarbon residue which can contain one to three halogen atoms and/or one to four heteroatoms, selected from the group consisting of nitrogen, oxygen and sulfur, it also being possible for $R^4$ and $R^5$, together with the nitrogen atom joining them, to form a 5- or 6-membered saturated or unsaturated heterocyclic radical, which optionally may also contain one or two further heteroatoms selected from the group consisting of nitrogen, oxygen and sulfur,

in general formula (II) and (IV) $R^1$ being H or $C_{1-17}$-hydrocarbon, preferably H, or $-CH_2-R^5$ with $R^5$ being H or $C_{1-16}$-hydrocarbon residue, which hydrocarbon can contain one to three halogen atoms and/or one to six heteroatoms, selected from the group consisting of nitrogen, oxygen and sulfur,

and preferably in general formula (II), (III) and (IV) $X^1$ and $X^2$ being S,

and at least one additional agrochemical agent, preferably selected from the group consisting of

- at least one further nitrification inhibitor, preferably selected from the group consisting of 2-(3,4-dimethyl-pyrazol-1-yl)-succinic acid (DMPSA), 3,4-dimethylpyrazole (DMP), 3,4-dimethylpyrazolephosphate (DMPP), dicyandiamide (DCD), 1H-1,2,4-triazole, 3-methylpyrazole (3-MP), 2-chloro-6-(trichloromethyl)-pyridine, 5-ethoxy-3-trichloromethyl-1,2,4-thiadiazol, 2-amino-4-chloro-6-methyl-pyrimidine, 2-mercapto-benzothiazole, 2-sulfanilamidothiazole, thiourea, sodium azide, potassium azide, 1-hydroxypyrazole, 2-methylpyrazole-1-carboxamide, 4-amino-1,2,4-triazole, 3-mercapto-1,2,4-triazole, 2,4-diamino-6-trichloromethyl-5-triazine, carbon bisulfide, ammonium thiosulfate, sodium trithiocarbonate, 2,3-dihydro-2,2-dimethyl-7-benzofuranol methyl carbamate and N-(2,6-dimethylphenyl)-N-(methoxyacetyl)-alanine methyl ester, AOA inhibitors or comammox inhibitors,

- at least one urease inhibitor, preferably selected from N-n-butylthiophosphoric triamide (NBTPT) and/or N-n-propylthiophosphoric triamide (NPTPT),

- at least one customary agrochemical auxiliary agent, preferably selected from the group consisting of aqueous and/or organic solvents, pH-adjusting agents, surfactants, wetting agents, spreading agents, adhesion promoters, carriers, fillers, viscosity-adjusting agents, emulsifiers, dispersants, sequestering agents, anti-settling agents, coalescing agents, rheology modifiers, defoaming agents, photo-protectors, anti-freeze agents, biostimulants, pesticides, biocides, plant growth regulators, safeners, penetrants, anticaking agents, mineral and/or vegetable oils and/or waxes, colorants and drift control agents,

and mixtures thereof.

A combination with at least one further nitrification inhibitor and/or urease inhibitor is preferred.

According to the present invention it was found that the N-heterocyclic compounds of general formula (I) and (II) and (III) and (IV) are strong new nitrification inhibitors, and target microorganisms and preferably ammonia-oxidizing bacteria (AOB) and possibly also ammonia-oxidizing archaea (AOA) and/or complete ammonia oxidation (comammox) bacteria like Candidatus Nitrospira kreftii. They can be combined with known nitrification inhibitors and/or urease inhibitors.

The new type of nitrification inhibitors is able to inhibit nitrification especially in soil.

Thiazolidine-thiols, and specifically the structural variants of these molecules of formula (I) and (II) and (III) and (IV), were found to be nitrification inhibitors. These nitrification inhibitors specifically inhibit ammonia oxidation in ammonia oxidizing bacteria, and effectively reduce ammonium losses in agricultural soils. They furthermore are Cu-chelators, and hence eliminate the action of Cu. Cu is necessary for the activity of the AMO enzyme complex, essential for ammonia oxidation. Therefore, the compounds inhibit nitrification because they chelate Cu.

According to the present invention, an N-heterocyclic compound of general formula (a) and/or (b), preferably of general formula (I) and/or (II) and/or (III) and/or (IV)

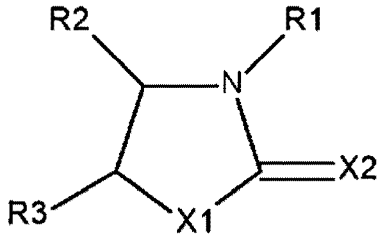
(I)

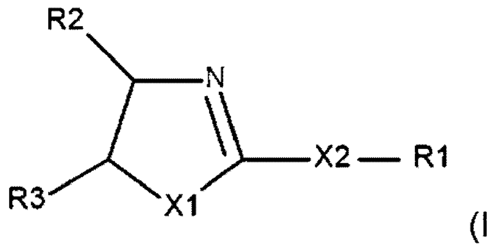
(II)

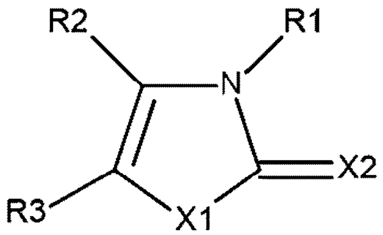
(III)

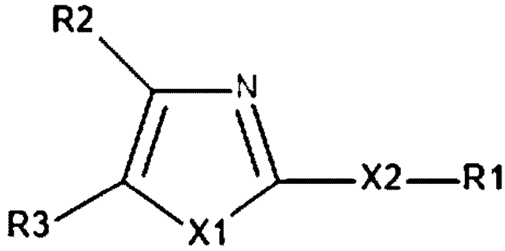
(IV)

with the following definitions:

$X^1$ being S or O, $X^2$ being S or O and at least one of $X^1$ and $X^2$ being S

$R^2$ H or $C_{1-4}$-alkyl,

$R^3$ H or $C_{1-4}$-alkyl

in general formula (I) and (III) $R^1$ being H, $C_{1-12}$-alkyl or $-CH_2-NR^4R^5$ with

$R^4$ hydrogen or $C_{1-4}$-alkyl,

$R^5$ $C_{1-12}$-hydrocarbon residue which can contain one to three halogen atoms and/or one to four heteroatoms, selected from the group consisting of nitrogen, oxygen and sulfur, it also being possible for $R^4$ and $R^5$, together with the nitrogen atom joining them, to form a 5- or 6-membered saturated or unsaturated heterocyclic radical, which optionally may also contain one or two further heteroatoms selected from the group consisting of nitrogen, oxygen and sulfur,

in general formula (II) and (IV) $R^1$ being H or $C_{1-17}$-hydrocarbon, preferably H, or $-CH_2-R^5$ with $R^5$ being H or $C_{1-16}$-hydrocarbon residue, which hydrocarbon can contain one to three halogen atoms and/or one to six heteroatoms, selected from the group consisting of nitrogen, oxygen and sulfur,

is employed as nitrification inhibitor, preferably in combination with a fertilizer, more preferably an (ammonium) nitrogen-containing fertilizer, e.g. solid or liquid inorganic, organic and/or organomineral fertilizer, or manure. The heterocyclic compound is for example employed as nitrification inhibitor on solid fertilizers, or is employed as nitrification inhibitor or as nitrogen stabilizer in liquid organic or inorganic or organomineral fertilizers or manure.

The N-heterocyclic compounds of general formula (I) and/or (II) and/or (III) and/or (IV) are mostly known *per se* and can be synthesized according to standard techniques. They partly are commercially available compounds and can be obtained from ENAMINE Ltd., UkrOrgSynthesis Ltd., or Vitas-M Laboratory, Ltd. or from Merck Millipore, Burlington, MA, USA, or Merck KGaA.

In general formula (I) and (II) and (III) and (IV) $X^1$ and $X^2$ can preferably be sulfur (S). In this case, the N-heterocyclic compounds are thiazolidine-2-thiol/thione compounds or compounds that can be easily cleaved to form thiazolidine-2-thiol compounds which are tautomeric with thiazolidine-2-thione compounds. The simplest molecule of this type is one in which in general formula (I) and (III) $X^1$ and $X^2$ are S and $R^1$, $R^2$ and $R^3$ are hydrogen. These compounds are shown in Examples 3 an 26 below.

If $X^1$ is oxygen (O) and $X^2$ is sulfur (S), the compounds are oxazolidine-2-thiol/thione compounds or compounds that can be easily cleaved to form oxazolidine-2-thiol compounds that are tautomeric with the oxazolidine-2-thione compounds.

When in general formula (I) $X^1$ is sulfur (S) and $X^2$ is oxygen (O), the compound is a thiazolidine-2-one compound.

Preferably, in general formula (I) and (II) and (III) and (IV) $X^2$ is S and $X^1$ is O or S, and more preferably $X^1$ and $X^2$ are S.

The compounds of general formula (II) are preferably thiazolidine-2-thioethers which can be cleaved to form thiazolidine-2-thiols and the tautomeric thiazolidine-2-thions.

Therefore, e.g. the compounds of general formula (I) and general formula (II) - and general formula (III) and (IV) in an analogous way - in which $X^1$ and $X^2$ are S are technically linked in that they are "capped" thiazolidine-thiol compounds or compounds containing a thiazolidine-thiol derived substructure. In both substructures the residue $R^1$ can be cleaved, leading to the thiazolidine-thiol compound.

Most preferred are the compounds of the general formula (I) and (II) and (III) and (IV) in which $X^1$ and $X^2$ are S.

These compound classes are potent nitrification inhibitors, preferably against AOB, and possibly also AOA and/or comammox.

In the compounds of the general formula (I) and (II) and (III) and (IV), some of the residues are hydrocarbon residues. Hydrocarbon residues are constituted of hydrogen and carbon atoms. They may be saturated, unsaturated or aromatic. Furthermore, they can contain the heteroatoms as identified above. The hydrocarbon residues can be linear, branched, cyclic, or can contain at least one linear or branched residue and at least one cyclic residue in the same structure.

If more than one heteroatom is present in the structure, heteroatoms of the same type are not directly covalently linked with each other. Preferably, heteroatoms are not directly covalently linked with other heteroatoms, but they intersect the hydrocarbon residue, with the exception being sulfonamides and sulfonic acid groups. Therefore, the heteroatoms are preferably non-adjacent.

Therefore, in $R^5$, heteroatoms of the same chemical element are not adjacent. Preferably, in $R^5$, heteroatoms are not directly linked to each other.

In general formula (I) and (III), $R^5$ can contain at least one cyclic structure. Preferably $R^5$ contains a 5- or 6-membered cyclic structure which can be annellated to a second 5- or 6-membered cyclic structure. In total, the compound of general formula (I) and (III) contains the additional cyclic structure shown in formula (I) and (III).

The compound of general formula (I) and (III) preferably contains two, three or four cyclic structures that furthermore can be condensed or annellated or not. The cyclic structures are preferably 5- or 6-membered. The cyclic structure depicted in general formula (I) and (III) is one of the cyclic structures forming the compound of general formula (I).

In general formula (II) and (IV) $R^5$ can contain at least one cyclic structure, preferably no, one or two cyclic structures, most preferably one or two cyclic structures. If two cyclic structures are present in $R^5$, they can be condensed or annellated or not. The cyclic structures are preferably 5- or 6-membered. Therefore, preferably the compound of general formula (II) and (IV) contains in $R^5$ a 5- or 6-membered cyclic structure which can be annellated to a second 5- or 6-membered cyclic structure.

The compound of general formula (II) and (IV) preferably contains two or three cyclic structures, one of which is the cyclic structure depicted in general formula (II) and (IV).

The cyclic structures in $R^5$ of compounds (I) and (II) and (III) and (IV) can be carbocyclic or heterocyclic. In condensed or annellated structures, one or more of the cyclic structures can be heterocyclic.

Condensed or annellated cyclic structures are cyclic structures in which two cyclic structures share two chemical elements, preferably two carbon atoms, in their respective ring structures.

The cyclic structure depicted in general formula (I) or (II) or (III) or (IV) cannot be condensed with a further cyclic structure. Therefore, only if in addition to the cyclic structure depicted in general formula (I) or (II) or (III) or (IV) two cyclic structures are present, these two additional cyclic structures can be condensed. The additional cyclic structures can be saturated or unsaturated or aromatic. If two condensed cyclic structures are present, it is possible that one cyclic structure is aromatic and the other cyclic structure is non-aromatic.

If two additional cyclic structures are not condensed, they can be directly covalently linked with each other. Alternatively, they can be linked by a spacer which can contain carbon atoms, heteroatoms or both. For example, the spacer can be a $C_{1-6}$-alkylene group, a heteroatom selected from the group consisting of oxygen, sulfur and nitrogen, or the spacer can for example be an amido group -C(=O)-NH-. If the cyclic groups are linked by a nitrogen atom, this nitrogen is preferably -NH- or -NR- with R being $C_{1-4}$-alkyl, more preferably R being methyl or ethyl.

Cyclic structures can also be described as structural elements that form part of the compounds of general formula (I) and (II) and (III) and (IV).

Preferably, $R^2$ is hydrogen, methyl or ethyl. Preferably, $R^3$ is hydrogen, methyl or ethyl. Preferably, $R^4$ is hydrogen, methyl or ethyl.

Most preferably, $R^2$ and $R^3$ are hydrogen.

Most preferably, $R^4$ is methyl or ethyl.

Preferred are compounds of general formula (I), wherein in general formula (I) $R^5$ is a $C_{3-10}$-hydrocarbon residue which can contain one to two halogen atoms and/or one to three heteroatoms and which contains at least one cyclic

structure.

More preferred are compounds of general formula (I), wherein in general formula (I) $R^5$ is a $C_{3-8}$-hydrocarbon residue which can contain one to two halogen atoms and/or one to three heteroatoms and which contains a 5- or 6-membered cyclic structure which can be annellated to a second 5- or 6-membered cyclic structure.

Preferred are compounds of general formula (II), wherein in general formula (II) $R^1$ is $C_{1-4}$-alkyl or $-CH_2-R^5$ with $R^5$ $C_{3-14}$-hydrocarbon residue, which can contain one or two halogen atoms and/or two to six heteroatoms, selected from the group consisting of nitrogen, oxygen and sulfur.

More preferred are compounds of general formula (II), wherein in general formula (II) $R^1$ is methyl, ethyl or $-CH_2-R^5$ with $R^5$ $C_{5-12}$-hydrocarbon residue, which can contain one halogen atom and/or two to four heteroatoms, selected from the group consisting of nitrogen, oxygen and sulfur. Halogen atoms are preferably selected from F, Cl and Br, more preferably from F and Cl. The residue $R^5$ can contain halogen atoms in combination with heteroatoms or as an alternative to heteroatoms.

In an additional embodiment, $R^4$ and $R^5$ in general formula (I) together with a nitrogen atom joining them form a 5- or preferably 6-membered saturated or unsaturated heterocyclic radical. In this embodiment, preferably $R^4$ and $R^5$ together with a nitrogen atom joining them form a 5- or preferably 6-membered saturated or unsaturated heterocyclic radical which optionally may also contain one further heteroatom selected from the group consisting of nitrogen and oxygen, and which can be part of a $C_{4-16}$-hydrocarbon residue which itself can contain (in total) 1 to 4 heteroatoms selected from the group consisting of nitrogen, oxygen, sulfur and fluorine in addition to the nitrogen atom joining $R^4$ and $R^5$.

More preferably, $R^4$ and $R^5$ together with the nitrogen atom joining them can form the saturated or unsaturated structural element

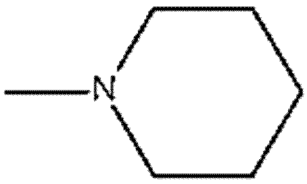

or

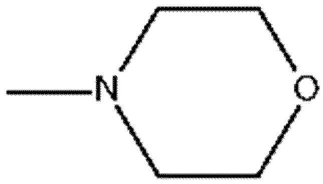

or

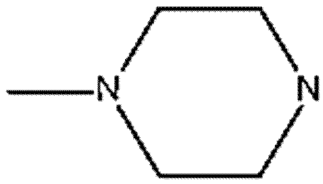

which can be part of a $C_{4-15}$-hydrocarbon residue which can contain 1 to 4 heteroatoms selected from the group consisting of nitrogen, oxygen, sulfur and fluorine in addition to the nitrogen atom joining $R^4$ and $R^5$. The depicted structures thus can contain C=C or C=N bonds or not.

They can be preferably part of a $C_{4-13}$-hydrocarbon residue, more preferably $C_{4-10}$-hydrocarbon residue, most preferably $C_{7-9}$-hydrocarbon residue. This hydrocarbon residue can contain 1 to 3 heteroatoms, more preferably can contain 1 or 2 heteroatoms selected from the group consisting of nitrogen, oxygen, sulfur and fluorine in addition to the nitrogen atom joining $R^4$ and $R^5$.

Saturated structural elements as depicted above are preferred.

According to one embodiment of the invention, the structural element or cyclic structure can contain one or two additional linkages on the structural element or cyclic structure to carbon atoms. Therefore, the structural element or cyclic structure can contain one or two substituents which are linked with the structural element or cyclic structure via a carbon atom. These one or two substituents together with a structural element or cyclic structure form the $C_{4-16}$-hydrocarbon residue, preferably $C_{4-13}$-hydrocarbon residue, more preferably $C_{4-10}$-hydrocarbon residue, specifically $C_{7-9}$-hydrocarbon residue.

Preferably, the structural element depicted above can contain one or two linkages to further carbon atoms in the meta- or para-position to the nitrogen of the $NR^4R^5$ group. More preferably, the structural element contains no further substituents in addition to the one or two linkages to further carbon atoms in the meta- or para-position to the nitrogen of the $NR^4R^5$ group.

Preferably, $R^4$ and $R^5$ are each linked to the N in $NR^4R^5$ via a carbon atom.

In the examples, specific residues $R^1$, $R^2$, $R^3$, $X^1/X^2$, $R^4$ and $R^5$ are disclosed for formula (I) and (II). Each of these residues can be employed for limiting the compounds of general formula (I) and (II), irrespective of the other residues. Thus, the compounds of general formula (I) and (II) can contain one or more of the residues $R^1$, $R^2$, $R^3$, $R^4$, $R^5$, $X^1$ and $X^2$ as disclosed in each of the examples.

In the compounds of formula (III) and (IV), preferably $R^1$ is hydrogen, so that the compounds of formula (III) and (IV) are tautomeric forms of one single compound. Preferably, in the compounds of formula (III) and (IV), $R^2$ and $R^3$ are hydrogen, methyl or ethyl, more preferably hydrogen or methyl, specifically hydrogen. Therefore, in the most preferred embodiment of the compounds of formula (III) and (IV), $X^1$ and $X^2$ are S and $R^1$, $R^2$ and $R^3$ are hydrogen.

Alkyl residues can be linear or branched.

Cyclic groups in the residues are preferably 5- or 6-membered rings which can be saturated, unsaturated or

aromatic. The rings can be purely hydrocarbon, e.g. phenyl groups. They can also contain nitrogen, oxygen and/or sulfur atoms as heteroatoms, preferably nitrogen or sulfur atoms. Examples of those residues are identified in the examples below.

The compounds of the general formula (I) and (II) and (III) and (IV) are nitrification inhibitors which inhibit the ammonia-oxidizing bacteria (AOB) and possibly also ammonia-oxidizing archaea (AOA), specifically *Nitrosomonas europaea* and/or *Nitrosospira multiformis.*

The nitrification inhibitor of the present invention is notable in particular for the fact that it effectively inhibits the nitrification of ammonium nitrogen in the soil over a long period of time.

Compounds that enable the formation of a thiol tautomer are highly active and preferred. For example, the 3H-1,3-thiazole-2-thione tautomers conform to general formula (III) and (IV) respectively, wherein $R^1$ to $R^3$ are hydrogen and $X^1$ and $X^2$ are sulfur. This includes the compounds having a labile aminomethyl linker or an H on the N in the 5-ring. The occupation of the N in the 5-ring prevents possible thiol formation, and also reduces nitrification inhibition. Similar, absence of the thion-group reduces activity. Side groups on the carbons of the thiazol 5-ring do not or hardly affect the activity. Furthermore, the most essential substructure, that is 1,3-thiazol-2-thione or is derived from 2-thiazoline-2-thiol, shows strong nitrification inhibition in soil as well.

The respective amount of each tautomer (equilibrium concentration) can be shifted by e.g. adjusting the pH of a solution of the compound to a desired value.

Furthermore, a superior nitrification inhibitory activity was found for molecules containing a thiazolidine-thiol derived substructure. These conform to formula (II) and are preferably thiazolidine-2-thioethers.

The nitrification inhibitors are described as those of general formula (I) and (II) and (III) and (IV). One or more compounds of formula (I) or (II) or (III) or (IV) can be employed as nitrification inhibitor and in the fertilizer discussed below. Furthermore, it is possible to employ mixtures of one or more compounds of general formula (I) and one or more compounds of general formula (II), as well as mixtures of one or more compounds of at least two of general formula (I) to (IV). This is reflected by the expression formula (I) and/or (IV) and/or (III) and/or (IV).

According to one embodiment of the invention, the compounds No 1-12 shown in the table on page 71 of WO 2020/020765 are excluded from the N-heterocyclic compounds to be employed. Furthermore, the compounds No 1-11 disclosed in WO 2020/020777 preferably are excluded from the N-heterocyclic compounds.

Preferably, in formula (I), compounds with $X^1$ and $X^2$ being S and $R^1$ being H or $CH_3$ or C (=O) hydrocarbyl are excluded. Hydrocarbyl in this case is a $C_{1-20}$-hydrocarbon residue, which can contain 1 to 3 halogen atoms and/or 1 to 4 hetero atoms, selected from the group consisting of nitrogen, oxygen and sulfur.

Preferably, the compounds of general formula (I), $R^1$ is not hydrogen, $C_{1-6}$-alkyl and preferably is not hydrogen or $C_{1-12}$-alkyl. Therefore, in general formula (I), $R^1$ is preferably $-CH_2-NH^4R^5$.

In formula (II), $R^1$ preferably is not hydrogen, methyl, ethyl, propagyl, C(=O)NH(phenyl), $CH_2C(=O)(phenyl)$ $H^+Br^-$, 2-methyl-4-chloro-phenyl, $C(=O)NH(CH_2CH_3)$.

$CH_2C(=O)OCH_3$ or $C(=S)N(CH_3)_2$.

Preferably, $R^1$ in general formula (I) is none of the residues disclosed for $R^N$ in WO 2020/020765, and $R^1$ in general formula (II) is none of the residues disclosed for $R^{sS}$ in WO 2020/020777.

The above disclaimers all refer to compounds in which $X^1$ and $X^2$ are sulfur.

According to one embodiment of the invention, the fertilizer mixtures do not contain oxazolidinthione, in contrast to CN 103 524 159 A.

It is expected that the nitrification inhibitor of the present invention possesses favorable toxicological properties, has a low vapor pressure, and is sorbed well in the soil. As a consequence, the nitrification inhibitor is neither emitted to the atmosphere by sublimation to any significant extent nor is easily leached by water. As a result, first of all, economic advantages arise, such as a high profitability in view of the longer-lasting effect of the nitrification inhibitor, and environmental advantages, such as a reduction in the burdening of air (climate gas-reducing) and of surface waters and ground water.

The nitrification inhibitors can be applied to soils or substrates which are fertilized with an inorganic or organic or organomineral fertilizer. Typically, they are employed in a fertilizer mixture comprising a (preferably inorganic) fertilizer and the N-heterocyclic compound of general formula (I) and/or (II) and/or (III) and/or (IV). Typically, the N-heterocyclic compound of general formula (I) and/or (II) and/or (III) and/or (IV) is employed in an amount of 10 to 10000 ppm by weight, more preferably 100 to 10000 ppm by weight, based on the (preferably inorganic) fertilizer without water. The application amount is based on the dry fertilizer.

The nitrification inhibitor according to the present invention can be employed in substance, in solution, dispersion or emulsion. Therefore, the invention also relates to a solution, dispersion or emulsion containing the N-heterocyclic compound of general formula (I) and/or (II) and/or (III) and/or (IV) of the present invention preferably in an amount of from 0.1 to 50 wt%, more preferably 0.5 to 30 wt%, most preferably 1 to 20 wt%.

Preferably, according to the present invention, inorganic fertilizers are employed for forming a fertilizer mixture, containing compounds A and B

A. an inorganic and/or organic and/or organomineral fertilizer and

B. 10 to 10000 weight-ppm, more preferably 100 to 10000 weight-ppm, based on the preferably inorganic fertilizer, of the N-heterocyclic compound of general formula (I) and/or (II) and/or (III) and/or (IV) as defined above.

The water fraction in compound A and in the fertilizer mixture is often not more than 1.5 wt%, preferably not more than 1.0 wt%, more preferably not more than 0.5 wt%, most preferably not more than 0.3 wt%, and is therefore negligible in the balance of quantities. Compounds A and B preferably make up at least 95 wt%, more preferably at least 98 wt% of the fertilizer mixture.

The nitrogen content of component A (without water), is often at least 12 wt%, preferably at least 20 wt%, more preferably at least 22 wt%. For example, the nitrogen content may be 25 to 29 wt%, particularly 26 to 28 wt%. The nitrogen content can be divided between fast-acting nitrate nitrogen and slow-acting ammonium nitrogen.

The inorganic fertilizers preferably are ammonium- and/or urea-containing fertilizers, more preferably ammonium-containing fertilizers which can additionally contain urea.

Urea-containing fertilizers are further described in WO 2016/207210.

The fertilizers employed according to the present invention can be of natural or synthetic origin and are applied to soil or to plant tissues to supply one or more plant nutrients essential to the growth of plants. The fertilizers employed according to the present invention should provide at least nitrogen as nutrient. Further nutrients are for example K and P. Multinutrient fertilizers or complex fertilizers provide two or more nutrients. Inorganic fertilizers exclude carbon-containing materials except ureas. Organic fertilizers are usually plant- or animal-derived matter. Organomineral fertilizers (combination of inorganic and organic fertilizers) can be employed as well.

The main nitrogen-based straight fertilizer is ammonia or its solutions. Ammonia nitrate is also widely used. Urea is another popular source of nitrogen, having the advantage that it is solid and non-explosive. A further nitrogen-based fertilizer is calcium ammonium nitrate.

The main straight phosphate fertilizers are the superphosphates including single superphosphate, phosphogypsum and triple superphosphate. The main potassium-based straight fertilizer is muriate of potash (MOP).

The binary fertilizers are preferably NP or NK fertilizers which can be monoammonium phosphate (MAP) and diammonium phosphate (DAP).

NPK fertilizers are three-component fertilizers providing nitrogen, phosphorus and potassium. NPK fertilizers can be produced by mixing straight fertilizers as mentioned above in bulk or in each granule, as in Nitrophoska®. In some cases, chemical reactions can occur between the two or more components.

Besides the main constituents, like N, P and K, micronutrients (trace elements) may be present in the fertilizers. The main micronutrients are molybdenum, zinc, boron and copper. These elements are typically provided as water-soluble salts.

Preferred fertilizers contain ammonium or urea. Examples of preferred ammonium-containing fertilizers are NPK fertilizers, calcium ammonium nitrate, ammonium sulfate nitrate, ammonium sulfate and ammonium phosphate.

Further preferred ingredients of the fertilizer compositions are for example trace elements, further minerals, standardizers, binders.

Organic fertilizers can describe those fertilizers with an organic or biologic origin, i.e. fertilizers derived from living or formerly living materials, like animals or plants or algae. Fertilizers of an organic origin include animal wastes, plant wastes e.g. from food processing or agriculture, compost, and treated sewage sludge (biosolids). Animal sources can be manures, but also products from the slaughter of animals, like blood meal, bone meal, feather meal, hides, hooves, and horns.

Soil amendments, like peat or coir, bark and sawdust can also be included.

Fertilizers can include without limitation, ammonium sulfate, ammonium nitrate, ammonium sulfate nitrate, ammonium chloride, ammonium bisulfate, ammonium polysulfide, ammonium thiosulfate, aqueous ammonia, anhydrous ammonia, ammonium polyphosphate, aluminum sulfate, calcium nitrate, calcium ammonium nitrate, calcium sulfate, calcined magnesite, calcitic limestone, calcium oxide, hampene (chelated iron), dolomitic limestone, hydrate lime, calcium carbonate, diammonium phosphate, monoammonium phosphate, potassium nitrate, potassium bicarbonate, monopotassium phosphate, magnesium nitrate, magnesium sulfate, potassium sulfate, potassium chloride, sodium nitrates, magnesian limestone, magnesia, disodium dihydromolybdate, cobalt chloride hexahydrate, nickel chloride hexahydrate, indole butyric acid, L-tryptophan, urea, urea-formaldehydes, urea ammonium nitrate, sulfur-coated urea, polymer-coated urea, isobutylidene diurea, $K_2SO_4$-$2MgSO_4$, kainite, sylvinite, kieserite, Epsom salts, elemental sulfur, marl, ground oyster shells, fish meal, oil cakes, fish manure, blood meal, rock phosphate, super phosphates, slag, bone meal, wood ash, biochar, algae, algae extract, struvite, manure, bat guano, peat moss, compost, green sand, cottonseed meal, feather meal, crab meal, fish emulsion or a combination thereof. The micronutrient fertilizer material can comprise boric acid, a borate, a boron frit, copper sulfate, a copper frit, a copper chelate, a sodium tetraborate decahydrate, an iron sulfate, an iron oxide, iron ammonium sulfate, an iron frit, an iron chelate, a manganese sulfate, a manganese oxide, a manganese

chelate, a manganese chloride, a manganese frit, a sodium molybdate, molybdic acid, a zinc sulfate, a zinc oxide, a zinc carbonate, a zinc frit, zinc phosphate, a zinc chelate or a combination thereof. In a particular embodiment, said fertilizer or fertilizer composition does not comprise insoluble selenium, selenium mineral, soluble selenium or salts thereof.

The treated (inorganic, organic or organomineral) fertilizers according to the invention are preferably present in powder form, prill form or in granule form.

Besides the N-heterocyclic compound of the general formula (I) and/or (II), formulations comprising the compound and agronomical adjuvants can be used for including the nitrification inhibitor in the fertilizer. Agronomical adjuvants are, for example, solvents, dispersants, pH-adjusting agents, fillers, stability improvers, surfactants.

The nitrification inhibitor can be included in the fertilizer mixture by mixing it or the formulation containing it with a solid or liquid fertilizer or fertilizer formulation. Preferably, the fertilizer mixture is in solid form and the nitrification inhibitor is applied to the surface of the (inorganic, organic or organomineral) fertilizer.

In a process for producing the fertilizer mixture of the present invention, the nitrification inhibitor or the formulation containing it can be introduced into the (inorganic, organic or organomineral) fertilizer and/or applied to the surface of the inorganic fertilizer.

Granules of fertilizers are impregnated or coated with the nitrification inhibitor, for example by being sprayed with a formulation like a solution or a dispersion of the nitrification inhibitor and subsequent drying. The method is known, for example, from DE-A-41 28 828. The sealing of the impregnated granules with, for example, a paraffin wax, which is an additional proposal in the latter document, is possible, but generally unnecessary.

Granulating assistants which can be employed for preparing solid fertilizer compositions can be lime, gypsum, silicon dioxide or kaolinite.

An alternative is the addition of the nitrification inhibitor during the actual production of the fertilizer, in the slurry, for example.

As a rule, nitrification inhibitors are customarily applied to the soil in amounts of 100 g/ha to 10 kg/ha. Preferably, the amount is in the range of from 300 g/ha to 5 kg/ha.

Delivery of the nitrification inhibitor in liquid fertilizer formulations may be accomplished, for example, by fertigation with or without excess water as described in DE-C-102 30 593.

The fertilizer mixture can contain at least one further nitrification inhibitor. Preferably, this at least one further nitrification inhibitor is inhibiting ammonia-oxidizing bacteria (AOB) and is preferably selected from the group consisting of 2-(3,4-dimethyl-pyrazol-1-yl)-succinic acid, 3,4-dimethylpyrazole (DMP), 3,4-dimethylpyrazolephosphate (DMPP), dicyandiamide (DCD), 1H-1,2,4-triazole, 3-methylpyrazole (3-MP), 2-chloro-6-(trichloromethyl)-pyridine, 5-ethoxy-3-trichloromethyl-1,2,4-thiadiazol, 2-amino-4-chloro-6-methyl-pyrimidine, 2-mercapto-benzothiazole, 2-sulfanilamidothiazole, thiourea, sodium azide, potassium azide, 1-hydroxypyrazole, 2-methylpyrazole-1-carboxamide, 4-amino-1,2,4-triazole, 3-mercapto-1,2,4-triazole, 2,4-diamino-6-trichloromethyl-5-triazine, carbon bisulfide, ammonium thiosulfate, sodium tri-thiocarbonate, 2,3-dihydro-2,2-dimethyl-7-benzofuranol methyl carbamate and N-(2,6-dimethylphenyl)-N-(methoxyacetyl)-alanine methyl ester. Also nitrification inhibitors inhibiting ammonia-oxidizing archaea (AOA) can be employed together with the N-heterocyclic compound of general formula (I).

When the additional nitrification inhibitor is employed, the weight ratio of the N-heterocyclic compound of general formula (I) and/or (II) and/or (III) and/or (IV) to the further nitrification inhibitor is preferably 0.1 to 10 : 1, more preferably 0.2 to 5 : 1, most preferably 0.5 to 2 : 1.

Furthermore, the fertilizer mixture can contain at least one urease inhibitor, which is preferably selected from N-n-butylthiophosphoric triamide (NBTPT) and/or N-n-propylthiophosphoric triamide (NPTPT). A urease inhibitor is typically added when the fertilizer contains urea. Urea nitrogen is liberated as ammonium by the action of urease, and the ammonium can undergo nitrification. Therefore, it can be advantageous to combine a urease inhibitor with the nitrification inhibitor.

If the N-heterocyclic compound of general formula (I) and/or (II) and/or (III) and/or (IV) of the present invention is combined with N-n-butylthiophosphoric triamide (NBTPT) and/or N-n-propylthiophosphoric triamide (NPTPT), the weight ratio of nitrification inhibitor(s) to urease inhibitor is preferably in the range of from 0.1 to 10 : 1, more preferably 0.5 to 8 : 1, most preferably 1 to 6 : 1.

Thiophosphoric triamides are known to be relatively easily converted to the corresponding phosphoric triamides and thiophosphoric diamides as well as other metabolites. Since, generally speaking, moisture cannot be entirely excluded, thiophosphoric triamide and the corresponding phosphoric triamide are frequently present in a mixture with one another. In this specification, therefore, the term "(thio)phosphoric triamide" identifies not only the pure thiophosphoric triamides and phosphoric triamides, respectively, but also mixtures thereof.

According to the present invention, also mixtures of N-(n-butyl)thiophosphoric triamide and N-(n-)propylthiophosphoric triamide can be employed, as described in EP-A-1 820 788.

The fertilizer mixtures can contain other ingredients, like coatings, for example of inorganic or organic polyacids, which are described in US 6,139,596.

Furthermore, coatings of powders, prills and granules can be formed of inorganic material, such as sulfur- or

mineral-based coatings, or with an organic polymer. Respective coatings are described in WO 2013/121384 on page 23, line 37 to page 24, line 16.

As stated above, the agrochemical formulations comprising the compounds of formula (I) and/or (II) are used in "effective amounts". This means that they are used in a quantity which allows to obtain the desired effect which is a (synergistic) increase of the health of a plant but which does not give rise to any phytotoxic symptom on the treated plant.

For use according to the present invention, the agrochemical formulations comprising the compounds of formula (I) and/or (II) and/or (III) and/or (IV) can be converted into the customary formulations, for example solutions, emulsions, suspensions, dusts, powders, pastes and granules. The use form depends on the particular intended purpose; in each case, it should ensure a fine and even distribution of the agrochemical formulations comprising the compounds of formula (I) and/or (II) and/or (III) and/or (IV) according to the present invention. The formulations are prepared in a known manner to the person skilled in the art.

The agrochemical formulations may also comprise auxiliaries which are customary in agrochemical formulations. The auxiliaries used depend on the particular application form and active substance, respectively. Examples for suitable auxiliaries are solvents, solid carriers, dispersants or emulsifiers (such as further solubilizers, protective colloids, surfactants and adhesion agents), organic and inorganic thickeners, bactericides, anti-freezing agents, anti-foaming agents, if appropriate colorants and tackifiers or binders (e.g. for seed treatment formulations).

Suitable solvents are water, organic solvents such as mineral oil fractions of medium to high boiling point, such as kerosene or diesel oil, furthermore coal tar oils and oils of vegetable or animal origin, aliphatic, cyclic and aromatic hydrocarbons, e.g. toluene, xylene, paraffin, tetrahydronaphthalene, alkylated naphthalenes or their derivatives, alcohols such as methanol, ethanol, propanol, butanol and cyclohexanol, glycols, ketones such as cyclohexanone and gamma-butyrolactone, fatty acid dimethylamides, fatty acids and fatty acid esters and strongly polar solvents, e.g. amines such as N-methylpyrrolidone.

Solid carriers are mineral earths such as silicates, silica gels, talc, kaolins, limestone, lime, chalk, bole, loess, clays, dolomite, diatomaceous earth, calcium sulfate, magnesium sulfate, magnesium oxide, ground synthetic materials, fertilizers, such as, e.g. ammonium sulfate, ammonium phosphate, ammonium nitrate, ureas, and products of vegetable origin, such as cereal meal, tree bark meal, wood meal and nutshell meal, cellulose powders and other solid carriers.

Suitable surfactants (adjuvants, wetters, tackifiers, dispersants or emulsifiers) are alkali metal, alkaline earth metal and ammonium salts of aromatic sulfonic acids, such as ligninsulfonic acid, phenolsulfonic acid, naphthalenesulfonic acid, dibutylnaphthalene-sulfonic acid and fatty acids, alkylsulfonates, alkyl-arylsulfonates, alkyl sulfates, laurylether sulfates, fatty alcohol sulfates, and sulfated hexa-, hepta- and octadecanolates, sulfated fatty alcohol glycol ethers, furthermore condensates of naphthalene or of naphthalenesulfonic acid with phenol and formaldehyde, polyoxy-ethylene octylphenyl ether, ethoxylated isooctylphenol, octylphenol, nonylphenol, alkylphenyl polyglycol ethers, tributylphenyl polyglycol ether, tristearyl-phenyl polyglycol ether, alkylaryl polyether alcohols, alcohol and fatty alcohol/ethylene oxide condensates, ethoxylated castor oil, polyoxyethylene alkyl ethers, ethoxylated polyoxypropylene, lauryl alcohol polyglycol ether acetal, sorbitol esters, lignin-sulfite waste liquid and proteins, denatured proteins, polysaccharides (e.g. methylcellulose), hydrophobically modified starches, polyvinyl alcohols, polycarboxylates types, polyalkoxylates, polyvinylamines, polyvinylpyrrolidone and the copolymers therof. Examples for thickeners (i.e. compounds that impart a modified flowability to formulations, i.e. high viscosity under static conditions and low viscosity during agitation) are polysaccharides and organic and inorganic clays such as Xanthan gum.

A 'pesticide' is something that prevents, destroys, or controls a harmful organism ('pest') or dis-ease, or protects plants or plant products during production, storage and transport.

The term includes, amongst others: herbicides, fungicides, insecticides, acaricides, nematicides, molluscicides, rodenticides, growth regulators, repellents, rodenticides and biocides as well as plant protection products.

Plant protection products are 'pesticides' that protect crops or desirable or target plants. They are primarily used in the agricultural sector but also in forestry, horticulture, amenity areas and in home gardens. They contain at least one active substance and have one of the following functions:

- protect plants or plant products against pests/diseases, before or after harvest;
- influence the life processes of plants (such as substances influencing their growth, excluding nutrients);
- preserve plant products;
- destroy or prevent growth of undesired plants or parts of plants.

They may also contain other components including safeners and synergists.

An active substance is any chemical, plant extract, pheromone or micro-organism (including viruses), that has action against 'pests' or on plants, parts of plants or plant products.

The most common use of pesticides is in the form of plant protection products (PPPs).

The term 'pesticide' is often used interchangeably with 'plant protection product', however, pes-ticide is a broader term that also covers non plant/crop uses, for example biocides.

Biocides, like herbicides, bactericides, molluscicides, algicides, phytotoxicants, fungicides, and their mixtures can be added.

Bactericides may be added for preservation and stabilization of the formulation. Examples for suitable bactericides are those based on dichlorophene and benzylalcohol hemi formal (Proxel® from ICI or Acticide® RS from Thor Chemie and Kathon® MK from Rohm & Haas) and isothiazolinone derivatives such as alkylisothiazolinones and benzisothiazolinones (Acticide® M BS from Thor Chemie). Examples for suitable anti-freezing agents are ethylene glycol, propylene glycol, urea and glycerin. Examples for anti-foaming agents are silicone emulsions (such as e.g. Silikon® SRE, Wacker, Germany or Rhodorsil®, Rhodia, France), long chain alcohols, fatty acids, salts of fatty acids, fluoroorganic compounds and agrochemical formulations comprising the compounds of formula (I) or (II) thereof.

Suitable colorants are pigments of low water solubility and solvent-soluble, e.g. water-soluble, dyes.

Examples for adhesion promoters, like tackifiers or binders, are polyvinylpyrrolidons, polyvinylacetates, polyvinyl alcohols and cellulose ethers (Tylose®, Shin-Etsu, Japan).

Granules, e.g. coated granules, impregnated granules and homogeneous granules, can be prepared by binding the active substances to solid carriers. Examples of solid carriers are mineral earths such as silica gels, silicates, talc, kaolin, attaclay, limestone, lime, chalk, bole, loess, clay, dolomite, diatomaceous earth, calcium sulfate, magnesium sulfate, magnesium oxide, ground synthetic materials, fertilizers, such as, e.g., ammonium sulfate, ammonium phosphate, ammonium nitrate, ureas, and products of vegetable origin, such as cereal meal, tree bark meal, wood meal and nutshell meal, cellulose powders and other solid carriers.

Anticaking agents like oils and/or waxes can be added.

The agrochemical formulations generally comprise between 0.01 and 95%, preferably between 0.1 and 90%, most preferably between 0.5 and 90%, by weight of active substances. The compounds of the agrochemical formulations comprising the compounds of formula (I) are employed in a purity of from 90% to 100%, preferably from 95% to 100% (according to their NMR spectrum).

The compounds of the agrochemical formulations comprising the compounds of formula (I) or (II) or (III) or (IV) can be used as such or in the form of their agricultural compositions, e.g. in the form of directly sprayable solutions, powders, suspensions, dispersions, emulsions, oil dispersions, pastes, dustable products, materials for spreading, or granules, by means of spraying, atomizing, dusting, spreading, brushing, immersing or pouring. The application forms depend entirely on the intended purposes; it is intended to ensure in each case the finest possible distribution of the compounds present in the agrochemical formulations comprising the compounds of formula (I) or (II) or (III) or (IV).

Aqueous application forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetter, tackifier, dispersant or emulsifier. Alternatively, it is possible to prepare concentrates composed of active substance, wetter, tackifier, dispersant or emulsifier and, if appropriate, solvent or oil, and such concentrates are suitable for dilution with water.

The active substance concentrations in the ready-to-use preparations can be varied within relatively wide ranges. In general, they are from 0.0001 to 10%, preferably from 0.001 to 1%, by weight of compounds of the agrochemical formulations comprising the compounds of formula (I) or (II) or (III) or (IV).

The compounds of the agrochemical formulations comprising the compounds of formula (I) may also be used successfully in the ultra-low-volume process (ULV), it being possible to apply compositions comprising over 95% by weight of active substance, or even to apply the active substance without additives.

Various types of oils, wetters, adjuvants, herbicides, fungicides, other pesticides, or bactericides may be added to the active compounds, if appropriate not until immediately prior to use (tank mix). These agents can be admixed with the compounds of the agrochemical formulations comprising the compounds of formula (I) or (II) or (III) or (IV) in a weight ratio of 1:100 to 100:1, preferably 1:10 to 10:1.

Compositions of this invention may also contain fertilizers (such as ammonium nitrate, urea, potash, and superphosphate), phytotoxicants and plant growth regulators (plant growth amendments) and safeners. These may be used sequentially or in combination with the above-described compositions, if appropriate also added only immediately prior to use (tank mix). For example, the plant(s) may be sprayed with a composition of this invention either before or after being treated with the fertilizers.

In the agrochemical formulations comprising the compounds of formula (I) or (II) or (III) or (IV), the weight ratio of the compounds generally depends from the properties of the compounds of the agrochemical formulations comprising the compounds of formula (I) or (II) or (III) or (IV).

The compounds of the agrochemical formulations comprising the compounds of formula (I) or (II) or (III) or (IV) can be used individually or already partially or completely mixed with one another to prepare the composition according to the invention. It is also possible for them to be packaged and used further as combination composition such as a kit of parts.

The user applies the composition according to the invention usually from a pre-dosage device, a knapsack sprayer, a spray tank or a spray plane. Here, the agrochemical composition is made up with water and/or buffer to the desired application concentration, it being possible, if appropriate, to add further auxiliaries, and the ready-to-use spray liquid or the agrochemical composition according to the invention is thus obtained. Usually, 50 to 500 liters of the ready-to-use spray liquid are applied per hectare of agricultural useful area, preferably 50 to 400 liters.

In a particular embodiment the absolute amount of the active compounds, represented by formula (I) or (II) or (III) or (IV), is used in a range between 1 mg/liter to 100 mg/liter, particularly in a range between 1 mg/l to 20 mg/l, particularly in a range between 1 mg/l to 25 mg/l, particularly in a range between 2 mg/l to 200 mg/l, particularly between 2 mg/l to 100 mg/l, particularly between 2 mg/l to 50 mg/l, particularly between 2 mg/l to 25 mg/l, particularly between 4 mg/l to 40 mg/l, particularly between 4 mg/l to 20 mg/l, particularly between 4 mg/l to 16 mg/l, particularly between 4 mg/l to 12 mg/l.

According to one embodiment, individual compounds of the agrochemical formulations comprising the compounds of formula (I) or (II) or (III) or (IV) formulated as composition (or formulation) such as parts of a kit or parts of the inventive mixture may be mixed by the user himself in a spray tank and further auxiliaries may be added, if appropriate (tank mix).

"Agrochemical", as used herein, means any active substance that may be used in the agrochemical industry (including agriculture, horticulture, floriculture and home and garden uses, but also products intended for non-crop related uses such as public health/pest control operator uses to control undesirable insects and rodents, household uses, such as household fungicides and insecticides and agents, for protecting plants or parts of plants, crops, bulbs, tubers, fruits (e.g. from harmful organisms, diseases or pests); for controlling, preferably promoting or increasing, the growth of plants; and/or for promoting the yield of plants, crops or the parts of plants that are harvested (e.g. its fruits, flowers, seeds etc.).

An "agrochemical composition" as used herein means a composition for agrochemical use, as herein defined, comprising at least one active substance of a compound of formula (I), optionally with one or more additives favoring optimal dispersion, atomization, deposition, leaf wetting, distribution, retention and/or uptake of agrochemicals. As a non-limiting example such additives are diluents, solvents, adjuvants, surfactants, wetting agents, spreading agents, oils, stickers, viscosity-adjusting agents (like thickeners, penetrants), pH-adjusting agents (like buffering agents, acidifiers), anti-settling agents, anti-freeze agents, photo-protectors, defoaming agents, biocides and/or drift control agents.

A "carrier", as used herein, means any solid, semi-solid or liquid carrier in or on(to) which an active substance can be suitably incorporated, included, immobilized, adsorbed, absorbed, bound, encapsulated, embedded, attached, or comprised. Non-limiting examples of such carriers include nanocapsules, microcapsules, nanospheres, microspheres, nanoparticles, microparticles, liposomes, vesicles, beads, a gel, weak ionic resin particles, liposomes, cochleate delivery vehicles, small granules, granulates, nano-tubes, bucky-balls, water droplets that are part of an water-in-oil emulsion, oil droplets that are part of an oil-in-water emulsion, organic materials such as cork, wood or other plant-derived materials (e.g. in the form of seed shells, wood chips, pulp, spheres, beads, sheets or any other suitable form), paper or cardboard, inorganic materials such as talc, clay, microcrystalline cellulose, silica, alumina, silicates and zeolites, or even microbial cells (such as yeast cells) or suitable fractions or fragments thereof.

The terms "effective amount", "effective dose" and "effective amount", as used herein, mean the amount needed to achieve the desired result or results. More exemplary information about amounts, ways of application and suitable ratios to be used is given below. The skilled artisan is well aware of the fact that such an amount can vary in a broad range and is dependent on various factors such as the treated cultivated plant as well as the climatic and soil conditions.

As used herein, the terms "determining", "measuring", "assessing", "monitoring" and "assaying" are used interchangeably and include both quantitative and qualitative determinations.

It is understood that the agrochemical composition is stable, both during storage and during utilization, meaning that the integrity of the agrochemical composition is maintained under storage and/or utilization conditions of the agrochemical composition, which may include elevated temperatures, freeze-thaw cycles, changes in pH or in ionic strength, UV-irradiation, presence of harmful chemicals and the like. More preferably, the compounds of formula (I), (II), (IIII), (IV) as herein described remain stable in the agrochemical composition, meaning that the integrity and the activity of the compounds are maintained under storage and/or utilization conditions of the agrochemical composition, which may include elevated temperatures, freeze-thaw cycles, changes in pH or in ionic strength, UV-irradiation, presence of harmful chemicals and the like. Most preferably, said compounds of formula (I), (II), (IIII), (IV) remain stable in the agrochemical composition when the agrochemical composition is stored at ambient temperature for a period of two years or when the agrochemical composition is stored at 54°C for a period of two weeks. Preferably, the agrochemical composition of the present invention retains at least about 70% activity, more preferably at least about 70% to 80% activity, most preferably about 80% to 90% activity or more. Examples of suitable carriers include, but are not limited to alginates, gums, starch, β-cyclodextrins, celluloses, polyurea, polyurethane, polyester, or clay.

The agrochemical composition may occur in any type of formulation, preferred formulations are powders, wettable powders, wettable granules, water dispersible granules, emulsions, emulsifiable concentrates, dusts, suspensions, suspension concentrates, suspoemulsions, capsule suspensions, aqueous dispersions, oil dispersions, aerosols, pastes, foams, slurries or flowable concentrates.

In yet another embodiment the invention provides the use of the agrochemical compositions of the invention for enhancing abiotic stress tolerance in plants.

The agrochemical composition according to the invention can be applied once to a crop, or it can be applied two or more times after each other with an interval between every two applications. The agrochemical composition according to the invention can be applied alone or in mixture with other materials, preferably other agrochemical compositions, to the crop; alternatively, the agrochemical composition according to the invention can be applied separately to the crop with other materials, preferably other agrochemical compositions, applied at different times to the same crop.

In yet another embodiment the invention provides a method for the manufacture of ('or the production of' which is equivalent wording) an agrochemical composition according to the invention, comprising formulating a molecule of formula (I) or (II) or (III) or (IV) as defined herein before, together with at least one customary agrochemical auxiliary agent. Suitable manufacturing methods are known in the art and include, but are not limited to, high or low shear mixing, wet or dry milling, drip-casting, encapsulating, emulsifying, coating, encrusting, pilling, extrusion granulation, fluid bed granulation, coextrusion, spray drying, spray chilling, atomization, addition or condensation polymerization, interfacial polymerization, in situ polymerization, coacervation, spray encapsulation, cooling melted dispersions, solvent evaporation, phase separation, solvent extraction, sol-gel polymerization, fluid bed coating, pan coating, melting, passive or active absorption or adsorption.

Customary agrochemical auxiliary agents are well-known in the art and preferably include, but are not limited to aqueous and/or organic solvents, pH-adjusting agents (like buffering agents, acidifiers), surfactants, wetting agents, spreading agents, adhesion promoters (like tackifiers, stickers), carriers, fillers, viscosity-adjusting agents (like thickeners), emulsifiers, dispersants, sequestering agents, anti-settling agents, coalescing agents, rheology modifiers, defoaming agents, photo-protectors, anti-freeze agents, biostimulants (including bacterial and/or fungal inoculants or microorganisms), biocides (preferably selected from herbicides, bactericides, phytotoxicants, fungicides, pesticides and mixtures thereof), plant growth regulators, safeners, penetrants, anticaking agents, mineral and/or vegetable oils and/or waxes, colorants and drift control agents or any suitable combination thereof.

The insecticide can include an organophosphate, a carbamate, a pyrethroid, an acaricide, an alkyl phthalate, boric acid, a borate, a fluoride, sulfur, a haloaromatic substituted urea, a hydrocarbon ester, a biologically-based insecticide, or a combination thereof. The herbicide, used to remove unwanted plants, can comprise a chlorophenoxy compound, a nitrophenolic compound, a nitrocresolic compound, a dipyridyl compound, an acetamide, an aliphatic acide, an anilide, a benzamide, a benzoic acid, a benzoic acid derivative, anisic acid, an anisic acid derivative, a benzonitrile, benzothiadiazinone dioxide, a thiocarbamate, a carbamate, carbanilate, chloropyridinyl, a cyclohexenone derivative, a dinitroaminobenzene derivative, a fluorodinitrotoluidine compound, isoxazolidinone, nicotinic acide, isopropylamine, an isopropylamine derivative, oxadiazolinone, a phosphate, a phthalate, a picolinic acid compound, a triazine, a triazole, a uracil, a urea derivative, endothall, sodium chlorate, or a combination thereof. The fungicide can comprise a substituted benzene, a thiocarbamate, an ethylene bis dithiocarbamate, a thiophthalidamide, a copper compound, an organomercury compound, an organotin compound, a cadmium compound, anilazine, benomyl, cyclohexamide, dodine, etridiazole, iprodione, metlaxyl, thiamimefon, triforine, or a combination thereof. The fungal inoculant can comprise a fungal inoculant of the family Glomeraceae, a fungal inoculant of the family Claroidoglomeraceae, a fungal inoculant of the family Acaulosporaceae, a fungal inoculant of the family Sacculospraceae, a fungal inoculant of the family Entrophosporaceae, a fungal inoculant of the family Pacidsproraceae, a fungal inoculant of the family Diversisporaceae, a fungal inoculant of the family Paraglomeraceae, a fungal inoculant of the family Archaeosporaceae, a fungal inoculant of the family Geosiphonaceae, a fungal inoculant of the family Ambisporacea, a fungal inoculant of the family Scutellosproaceae, a fungal inoculant of the family Dentiscultataceae, a fungal inoculant of the family Racocetraceae, a fungal inoculant of the phylum Basidiomycota, a fungal inoculant of the phylum Ascomycota, a fungal inoculant of the phylum Zygomycota, a fungal inoculant of the genus Glomus or a combination thereof. The bacterial inoculant can include a bacterial inoculant of the genus Rhizobium, bacterial inoculant of the genus Bradyrhizobium, bacterial inoculant of the genus Mesorhizobium, bacterial inoculant of the genus Azorhizobium, bacterial inoculant of the genus Allorhizobium, bacterial inoculant of the genus Burkholderia, bacterial inoculant of the genus Sinorhizobium, bacterial inoculant of the genus Kluyvera, bacterial inoculant of the genus Azotobacter, bacterial inoculant of the genus Pseudomonas, bacterial inoculant of the genus Azosprillium, bacterial inoculant of the genus Bacillus, bacterial inoculant of the genus Streptomyces, bacterial inoculant of the genus Paenibacillus, bacterial inoculant of the genus Paracoccus, bacterial inoculant of the genus Enterobacter, bacterial inoculant of the genus Alcaligenes, bacterial inoculant of the genus Mycobacterium, bacterial inoculant of the genus Trichoderma, bacterial inoculant of the genus Gliocladium, bacterial inoculant of the genus Klebsiella, or a combination thereof.

Also, the mixture can comprise additionally at least one microorganism selected from the list consisting of Bacillus subtilis strain 713, Bacillus amyloliquefaciens MBI 600, Bacillus pumillus QST2808, Pseudomonas fluorescens, Bradyrhizobium japonicum, Trichoderma vireus, Pseudomonas putida, Trichoderma harzianum Rifai strain T22, Penicillium bilaii, Mesorhizobium, Azospirillum, Azotobacter vinelandii and Clostridium pasteurianum, Glomus species.

The N-heterocyclic compounds of general formula (I) and/or (II) and/or (III) and/or (IV) employed according to the

present invention can be employed in combination with these auxiliaries. The auxiliaries used depend on the particular application form and the active substance and preferably include solvents, solid carriers, dispersants or emulsifiers, such as solubilizers, protective colloids, surfactants and adhesion agents. Furthermore, organic and inorganic thickeners, bactericides, anti-freezing agents, anti-foaming agents, if appropriate, colorants and tackifiers or binders can be employed in combination with the nitrification inhibitors and in the fertilizer mixture. Suitable auxiliaries are discussed in WO 2013/121384 on pages 25 to 26.

Further possible preferred ingredients are oils, wetters, adjuvants, biostimulants, herbicides, bactericides, other fungicides and/or pesticides. They are for example discussed in WO 2013/121384 on pages 28/29.

The fertilizer mixtures are preferably in solid form, including powders, prills and granules. Furthermore, it is possible to deliver the nitrification inhibitor in the form of a formulation, solution or dispersion separately or simultaneously with a fertilizer.

Furthermore, the nitrification inhibitor of the present invention can be used for reducing the nitrogen losses in organic fertilizers and also on harvest refuse and on grazed land or during the storage of liquid manure and following the ammonium load in animal stalls.

For respective applications, reference can be made to US 6,139,596 and WO 2013/121384 as well as WO 2015/086823 and WO 2016/207210.

The present invention also relates to a method of fertilizing soils exploited agriculturally or horticulturally, wherein a fertilizer mixture containing compounds A and B

A. an inorganic and/or organic and/or organomineral fertilizer and

B. 10 to 10000 weight-ppm, based on the inorganic fertilizer, of an N-heterocyclic compound of general formula (I) and/or (II) and/or (III) and/or (IV) as defined above,

or compounds A and B separately, but within a period of 0 to 5 hours, preferably 0 to 1 hour, more preferably approximately at the same time,

is applied to the soils.

In parallel with the improvement of the utilization of nitrogen in the ammonium- or urea-containing mineral, organic and organomineral fertilizers, the use of the nitrification inhibitors, according to the present invention, and of compositions containing them has the effect that there is an increase, in some cases considerably, in the yields and production of biomass of crop plants.

Equally, the nitrification inhibitor according to the invention may be added to organic fertilizers, such as liquid manure, for example, during the actual storage of such fertilizers in order to prevent nitrogen nutrient losses by virtue of a decelerated conversion of the individual forms of nitrogen into gaseous, therefore volatile, nitrogen compounds, and, at the same time, to contribute to a lowering of the ammonia load in animal stalls. Moreover, the nitrification inhibitor or compositions containing the nitrification inhibitor may be used on agricultural stovers and grazed land for the purpose of reducing gaseous nitrogen losses and for preventing instances of nitrate leaching.

Generally, the N-heterocyclic compound of the general formula (I) or (II) or (III) or (IV) can be used for reducing the nitrogen or carbon losses in inorganic and/or organic and/or organomineral fertilizers or nitrogen- or carbon-containing compounds or materials and also on harvest refuse and on grazed land or during the storage of liquid manure and for lowering the ammonia load in animal stalls.

Nitrogen losses are often due to N2O and/or NO emissions or NO3- leaching. Nitrogen losses can occur in nitrogen-containing compounds or materials, examples of which are roots, plants, fertilizers, animals, etc. Typically, nitrogen losses occur from nitrogen-containing mineral fertilizers or any organic nitrogen-containing matter. The term "nitrogen losses" encompasses all forms of nitrogen or nitrogen compounds which are lost via emissions or leaching, as indicated above. One example are greenhouse gas emissions that can be lowered by the use of the N-heterocyclic compound of the present invention.

The same is true for carbon losses which can occur in carbon-containing compounds or materials. Carbon-containing compounds or materials are for example carbonate-containing mineral fertilizers and carbon-containing organic matter, like roots, plants, animals, organic fertilizers, etc. Carbon losses often occur as CO2 emissions, which are part of the greenhouse gas emissions.

Therefore, the N-heterocyclic compounds of the present invention can be used for preventing or reducing greenhouse gas emissions from nitrogen- or carbon-containing compounds or materials. These materials often contain nitrogen and/or carbon in covalently or ionically bonded form, e.g. in the form of ammonium, protein, nitrate, carbonate, carbohydrates, cellulose, or other organic carbon-containing compounds. Thus, the terms "carbon" and "nitrogen" can refer to the elemental form or to compounds or materials containing carbon and/or nitrogen atoms.

Nitrogen- and/or carbon-containing compounds or materials can be present in fertilizers, in soil, or in the environment. The most prominent effect of the N-heterocyclic compounds of the present invention is the reduction of greenhouse gas emissions from soil containing nitrogen-containing compounds or materials.

Most efficient is the reduction of greenhouse gas emissions from fertilized soil. These greenhouse gas emissions are often caused by processes downstream of nitrification.

The plants to be treated or rooted in soil to be treated according to the invention are preferably selected from the group consisting of agricultural, silvicultural, ornamental and horticultural plants, each in its natural or genetically modified form. Preferably, non-transgenic agricultural plants are treated.

Preferred agricultural plants are field crops selected from the group consisting of potatoes, sugar beets, wheat, barley, rye, oat, sorghum, rice, maize, cotton, rapeseed, oilseed rape, canola, soybeans, peas, field beans, sunflowers, sugar cane; cucumbers, tomatoes, onions, leeks, lettuce, squashes; even more preferably the plant is selected from the group consisting of wheat, barley, oat, rye, soybean, maize, oilseed rape, cotton, sugar cane, rice and sorghum.

In a preferred embodiment of the invention, the plant to be treated is selected from the group consisting of tomato, potato, wheat, barley, oat, rye, soybean, maize, oilseed rape, canola, sunflower, cotton, sugar cane, sugar beet, rice, sorghum, pasture grass and grazed land.

In another preferred embodiment of the invention, the plant to be treated is selected from the group consisting of tomato, potato, wheat, barley, oat, rye, soybean, maize, oilseed rape, canola, sunflower, cotton, sugar cane, sugar beet, rice and sorghum.

In an especially preferred embodiment of the invention, the plants to be treated are selected from the group consisting of tomato, wheat, barley, oat, rye, maize, oilseed rape, canola, sugar cane, and rice.

In one embodiment, the plant to be treated according to the method of the invention is an agricultural plant. "Agricultural plants" are plants of which a part (e.g. seeds) or all is harvested or cultivated on a commercial scale or which serve as an important source of feed, food, fibres (e.g. cotton, linen), combustibles (e.g. wood, bioethanol, biodiesel, biomass) or other chemical compounds. Preferred agricultural plants are for example cereals, e.g. wheat, rye, barley, triticale, oats, sorghum or rice, beet, e.g. sugar beet or fodder beet; fruits, such as pomes, stone fruits or soft fruits, e.g. apples, pears, plums, peaches, almonds, cherries, strawberries, raspberries, blackberries or gooseberries; leguminous plants, such as lentils, peas, alfalfa or soybeans; oil plants, such as rapeseed, oilseed rape, canola, linseed, mustard, olives, sunflowers, coconut, cocoa beans, castor oil plants, oil palms, ground nuts or soybeans; cucurbits, such as squashes, cucumber or melons; fiber plants, such as cotton, flax, hemp or jute; citrus fruit, such as oranges, lemons, grapefruits or mandarins; vegetables, such as spinach, lettuce, asparagus, cabbages, carrots, onions, tomatoes, potatoes, cucurbits or paprika; lauraceous plants, such as avocados, cinnamon or camphor; energy and raw material plants, such as maize, soybean, rapeseed, canola, sugar cane or oil palm; tobacco; nuts; coffee; tea; bananas; vines (table grapes and grape juice grape vines); hop; turf; natural rubber plants.

Pasture grass and grassland are composed of grass or grass mixtures comprising for example Bluegrass (Poa spp.), Bentgrass (Agrostis spp.), Ryegrasses (Lolium spp.), Fescues (Festuca spp., hybrids, and cultivars), Zoysiagrass (Zoysia spp.), Bermudagrass (Cynodon spp.), St. Augustine grass, Bahiagrass (Paspalum), Centipedegrass (Eremachloa), Carpetgrass (Axonopus), Buffalograss and Grama grass. Pastures may be also composed of mixtures comprising afore mentioned grasses, for example Ryegrass, and Trifolium species, for example Trifolium pratensis and Trifolium repens, Medicago species like Medicago sativa, Lotus species like Lotus corniculatus, and Melilotus species, for example Melilotus albus.

In one embodiment, the plant to be treated according to the method of the invention is a horticultural plant. The term "horticultural plants" are to be understood as plants which are commonly used in horticulture - e.g. the cultivation of ornamentals, herbs, vegetables and/or fruits. Examples for ornamentals are turf, geranium, pelargonia, petunia, begonia and fuchsia. Examples for vegetables are potatoes, tomatoes, peppers, cucurbits, cucumbers, melons, watermelons, garlic, onions, carrots, cabbage, beans, peas and lettuce and more preferably from tomatoes, onions, peas and lettuce. Examples for fruits are apples, pears, cherries, strawberry, citrus, peaches, apricots and blueberries. In horticulture, often a substrate replaces (part of) the soil.

In one embodiment, the plant to be treated according to the method of the invention is an ornamental plant. "Ornamental plants" are plants which are commonly used in gardening, e.g. in parks, gardens and on balconies. Examples are turf, geranium, pelargonia, petunia, begonia and fuchsia.

In one embodiment, the plant to be treated according to the method of the invention is a silvicultural plant. The term "silvicultural plant" is to be understood as trees, more specifically trees used in reforestation or industrial plantations. Industrial plantations generally serve for the commercial production of forest products, such as wood, pulp, paper, rubber tree, Christmas trees, or young trees for gardening purposes. Examples for silvicultural plants are conifers, like pines, in particular Pinus spec, fir and spruce, eucalyptus, tropical trees, like teak, rubber tree, oil palm, willow (Salix), in particular Salix spec, poplar (cottonwood), in particular Populus spec, beech, in particular Fagus spec, birch, oil palm, and oak.

The following definitions apply:

The term "plants" is to be understood as plants of economic importance and/or men-grown plants. They are preferably

selected from agricultural, silvicultural, ornamental and horticultural plants, each in its natural or genetically modified form. The term "plant" as used herein includes all parts of a plant, such as germinating seeds, emerging seedlings, herbaceous vegetation, as well as established woody plants, including all below-ground portions (such as the roots) and aboveground portions.

The term "soil" is to be understood as a natural body comprised of living (e.g. microorganisms (such as bacteria and fungi), animals and plants) and non-living matter (e.g. minerals and organic matter (e.g. organic compounds in varying degrees of decomposition), liquid, and gases) that occurs on the land surface, and is characterized by soil horizons that are distinguishable from the initial material as a result of various physical, chemical, biological, and anthropogenic processes.

The term "nitrification inhibitor" is to be understood as any chemical substance which slows down or retards the nitrification process which is typically occurring in (fertilized) soil. Nitrification inhibitors retard the natural transformation of ammonium into nitrate and target microorganisms and preferably ammonia-oxidizing bacteria (AOB), preferably by inhibiting the activity of the bacteria, such as Nitrosomonas spp. and/or Nitrosospira spp. They may additionally act on ammonia-oxidizing archaea (AOA). The nitrification inhibitor is most often combined with a fertilizer, preferably an (ammonium) nitrogen-containing fertilizer, e.g. solid or liquid inorganic, organic and/or organomineral fertilizer, or manure.

The term "nitrification" is to be understood as the biological oxidation of ammonia (NH3) or ammonium ($NH_4^+$) with oxygen into nitrite (NO2-) followed by the oxidation of these nitrites into nitrates (NO3-) by microorganisms. Besides nitrate (NO3-) nitrous oxide is also produced though nitrification. Nitrification is an important step in the nitrogen cycle in soil.

The term "denitrification" is to be understood as the microbiological conversion of nitrate (NO3-) and nitrite (NO2-) to gaseous forms of nitrogen, generally N2 or N2O. This respiratory process reduces oxidized forms of nitrogen in response to the oxidation of an electron donor such as organic matter. The preferred nitrogen electron acceptors in order of most to least thermodynamically favorable include: nitrate (NO3-), nitrite (NO2-), nitric oxide (NO), and nitrous oxide (N2O). Within the general nitrogen cycle, denitrification completes the cycle by returning N2 to the atmosphere. The process is performed primarily by heterotrophic bacteria (such as Paracoccus denitrificans and various pseudomonads), although autotrophic denitrifiers have also been identified (e.g. Thiobacillus denitrificans). Denitrifiers are represented in all main phylogenetic groups. When faced with a shortage of oxygen many bacterial species are able to switch from using oxygen to using nitrates to support respiration in a process known as denitrification, during which the water-soluble nitrates are converted to gaseous products, including nitrous oxide, that are emitted into the atmosphere.

"Nitrous oxide", commonly known as happy gas or laughing gas, is a chemical compound with the chemical formula N2O. At room temperature, it is a colorless non-flammable gas. Nitrous oxide is produced naturally in soils through the microbial processes of nitrification and denitrification. These natural emissions of nitrous oxide can be increased by a variety of agricultural practices and activities including for example a) direct addition of nitrogen to soils by using mineral and organic fertilizers, b) growing of nitrogen-fixing crops, c) cultivation of high organic content soils.

The term "fertilizers" is to be understood as (chemical) compounds applied to promote plant and fruit growth. Fertilizers are typically applied either through the soil (for uptake by plant roots) or by foliar feeding (for uptake through leaves). The term "fertilizers" can be subdivided into two major categories: a) organic fertilizers (composed of decayed plant/animal matter) and b) inorganic fertilizers (composed of chemicals and minerals). Organic fertilizers include slurry, worm castings, peat, seaweed, sewage, and guano. Manufactured organic fertilizers include compost, blood meal, bone meal and seaweed extracts. Further examples are enzymatically digested proteins, fish meal, and feather meal. The decomposing crop residue from prior years and manure are another source of fertility. In addition, naturally occurring minerals, such as mine rock phosphate, sulfate of potash and limestone, are also considered as belonging to inorganic fertilizers. Inorganic fertilizers are usually manufactured through chemical processes (such as the Haber-Bosch process), also using naturally occurring deposits, while chemically altering them (e.g. concentrated triple superphosphate). Naturally occurring inorganic fertilizers include Chilean sodium nitrate, mine rock phosphate, and limestone. As a third category organomineral fertilizers can be mentioned, as a combination of inorganic and organic fertilizers.

The term "fertilizer comprising urea" (urea fertilizer) is defined as synthetic fertilizers comprising urea, excluding any naturally occurring fertilizers comprising urea (for instance manure as an example for a naturally occurring fertilizer comprising urea). Examples of fertilizer comprising urea are urea ammonium nitrate (UAN), isobutylidene diurea (IBDU), crotonylidene diurea (CDU) and urea formaldehyde (UF). Urea is usually made as granulated material or prills. Urea fertilizer can be produced by dropping the liquid urea from a prill tower while drying the product. Urea can also be obtained as a liquid formulation, which may be used for foliar application, e.g. on potatoes, wheat, vegetables and soybeans as well as liquid application to the field. It is commonly mixed with ammonium nitrate to form UAN with 28% N.

The term "locus" (plant habitat) is to be understood as any type of environment, soil, area or material where the plant is growing or intended to grow. Especially preferred according to the invention is soil.

The invention is further illustrated by the following examples which show that:

- The compounds of formula (I) and (II) and (III) and (IV) are strong nitrification inhibitors;

- New nitrification inhibitors show high potency and specificity;
- The new nitrification inhibitors act specifically on nitrifying bacteria and target the NH3 oxidation step in the nitrifying bacteria;
- The new nitrification inhibitors reduce ammonium consumption in soil.

**Examples**

**1. Heterocyclic compounds of general formula (I) to (IV)**

Different heterocyclic compounds of general formula (I) to (IV) were obtained from ENAMINE Ltd., UkrOrgSynthesis Ltd., or Vitas-M Laboratory, Ltd. The respective compounds are shown in Table 1 below. 2-Thiazoline-2-thiol is also available from Sigma-Aldrich.

The compounds of formula (I) and (II) and (III)/(IV) were tested for their nitrification inhibition effect in two screens, using assays with the nitrifying bacteria N. europaea and N. multiformis. In practice, ammonium and candidate nitrification inhibitors (at 100 μM) were added to a dense bacterial culture in multiwell culture plates and after 24h of incubation, the nitrite level was measured. To normalize for differences between batches of cultures and multiwell culture plates, for each well a relative nitrification was calculated that was normalized towards both the negative and positive controls (see "Material and Methods" below for full details).

**2. Material and methods**

**2.1 Nitrite measurements**

Nitrite (NO2-) was measured using the Griess reagent (Product number: G4410, Griess reagent (modified), Sigma-Aldrich). Equal volumes of the sample or a diluted sample and the Griess reagent were mixed a transparent flat bottom multi-well plate and incubated in the dark at room temperature for 10 minutes. Absorbance values at 540 nm were measured spectrophotometrically (EnVision, Perkin Elmer®) and used to calculate the [NO2-] by use of a standard curve.

**2.2 Ammonium measurements**

Ammonium ($NH_4^+$) was measured via a modified Berthelot's reagent protocol. 8 μl culture sample, 35 μl reagent A (0.5 g NaOH with 8 ml NaClO (2.5%) in 92 ml MilliQ) and 33 μl reagent B (1 g salicylic acid, 0.5 g NaOH and 1.0237 g sodium nitroprusside dihydrate in 100 ml MilliQ) were consecutively added to 160 μl MilliQ in flat bottom 96-well plates (Cat. No. 353072, Falcon® 96 Well Clear Microplate, Corning). Absorbance values at 635 nm were measured spectrophotometrically (EnVision, Perkin Elmer®) after a 30 min incubation and used to calculate [NH4+] by use of a standard curve.

**2.3 Culture maintenance**

**2.3.1 Nitrosomonas europaea**

Nitrosomonas europaea growth medium was prepared by aseptically combining 900 mL of stock solution 1 (27.75 mM (NH4)2SO4, 3.35 mM KH2PO4, 0.83 μM MgSO4, 0.22 μM CaCl2, 11 μM FeSO4, 18.33 μM EDTA and 0.56 μM CuSO4) with 100 mL of stock solution 2 (400 mM KH2PO4 and 40 mM NaH2PO4, pH 8.0 (NaOH)) and 8 mL stock solution 3 (5% anhydrous Na2CO3) solution. All three stock solutions were autoclaved in advance. Nitrosomonas europaea (ATCC□ 25978□) cells were grown in sterile Erlenmeyer flasks sealed with tape (Micropore™ Surgical Tape 1530-1, 3M™) at 28°C and shaken (± 150 rpm) in the dark. Cultures in the late-log phase ([NO2-] = 10 - 20 mM) were subcultivated by centrifugation (4000 rpm, 15 min, 5°C) and complete medium refreshment by discarding the supernatant and resuspension of the bacterial cell pellets in freshly prepared Nitrosomonas europaea growth medium.

**2.3.2 Nitrosospira multiformis**

Nitrosospira multiformis (NCIMB 11849) cells were grown in Erlenmeyer flasks filled with autoclaved 181-medium for AOB (NCIMB Ltd) that contained 1.78 mM (NH4)2SO4, 1.47 mM KH2PO4, 272 μM CaCl2 x 2 H2O, 162 μM MgSO4 x 7 H2O, 1 mL stock solution 1 (1.8 mM FeSO4 x 7 H2O and 1.49 mM NaEDTA) and 1 ml stock solution 2 (0,5% phenol red, pH indicator) (pH 7.5-8). pH was maintained by regular additions of sterile 5% Na2CO3. Cultures flasks were

sealed with tape (Micropore™ Surgical Tape 1530-1, 3M™) and incubated in the dark at 30°C while shaking (± 150 rpm). Late-log phase cultures ([NO2-] = ± 3 mM) were subcultivated by centrifugation (4000 rpm, 15 min, 5°C) and complete medium refreshment by discarding the supernatant and resuspension of the bacterial cell pellets in pH-adjusted 181-medium.

**Nitrification inhibition assays**

**2.4.1 Nitrosomonas europaea**

To prepare a high-throughput nitrification inhibition assay using N. europaea, late-log phase cultures were subcultivated two days before the assay. Just before the assay, the cultures were 5 times overconcentrated with fresh growth medium after centrifugation (4000 rpm, 15 min, 5°C) in 50 mL centrifuge tubes (Cat. No. 430829, CentriStar™ Conical Centrifuge Tubes, Corning®). Per batch, all the cultures were pooled in one sterile Schott bottle and subsequently dispensed into 384-well plates (Cat. No. 781086, CELLSTAR® plate, Greiner Bio-One; 50 μl/well) by use of a dispenser (Multidrop™ Combi Reagent Dispenser, Thermo Scientific™) that was first flushed with growth medium. Specifically for the high-throughput screen for new nitrification inhibitors, 0.5 μl of 99.99% DMSO (negative control - final concentration 1%) was added to the outer two columns on the left side of the plate and 0.5 μl of 10 mM DMP (3,4-dimethylpyrazole, positive control - final concentration 100 μM) was added to the outer two columns on the right side manually. Finally, 0.5 μl of candidate nitrification inhibitors (5 mM stock solutions in 99.99% DMSO - final concentration 50 μM) were added to the central wells using a pin tool on a Tecan robot (Freedom EVO®, Tecan). In between additions, the pins were washed in sequence with 99.5% DMSO, MilliQ water and 100% ethanol and air-dried. All plates were separately wrapped in Parafilm. Stacks of 4 plates were put on top of a 96-well plate filled with 100 μl MilliQ per well, covered with aluminum foil and shaken at 150 rpm at 28°C. 24 h later, NO2- production was assessed. For this, the samples were first 200 times diluted by pipetting 1.5 μl of samples into 300 μl of fresh growth medium in intermediate plates (Cat. No. 353077, Falcon® 96-well Clear Round Bottom Microplate, Corning®), to then mix 15 μl of the diluted samples with 15 μl Griess reagent in the wells of a transparent, flat bottom 384-well plate (Cat. No. X7001, Low Profile Microplate, Molecular Devices) that was measured spectrophotometrically at 540 nm (EnVision, Perkin Elmer®).

**2.4.2 Nitrosospira multiformis**

500 mL cultures grown in 1-L Erlenmeyer flasks that reached the late-log phase ([NO2-] ≥ 3 mM) within 3 to 4 days and showed ≥ 500 μM increase in [NO2-] over the last 24h were used for a high-throughput nitrification inhibition assay. Cultures were first 5 times overconcentrated in fresh 181-medium by centrifugation (4000 rpm, 15 min, 5°C) in 50 mL centrifuge tubes (Cat. No. 430829, CentriStar™ Conical Centrifuge Tubes, Corning®). Per batch, all the cultures were pooled in one sterile Schott bottle and subsequently dispensed in 384-well plates (Cat. No. 781086, CELLSTAR® plate, Greiner Bio-One; 50 μl/well) by use of a dispenser (Multidrop™ Combi Reagent Dispenser, Thermo Scientific™) that was first flushed with 181-medium. 0.5 μl of 99.99% DMSO (negative control - final concentration 1%) was added to the outer two columns on the left side of the plate and 0.5 μl of 10 mM DMP (3,4-dimethylpyrazole, positive control - final concentration 100 μM) was added to the outer two columns on the right side manually. Finally, 0.5 μl of candidate nitrification inhibitors (5 mM stock solutions in 99.99% DMSO - final concentration 50 μM) were added to the central wells using a pin tool on a Tecan robot (Freedom EVO®, Tecan). In between additions, the pins were washed in sequence with 99.5% DMSO, MilliQ water and 100% ethanol and air-dried. All plates were separately wrapped in Parafilm. Stacks of 4 plates were put on top of a 96-well plate filled with 100 μl MilliQ per well, covered with aluminum foil and shaken (± 150 rpm) at 30°C. 24 h later, NO2- production was assessed. For this, the samples were first 100 times diluted by pipetting 3 μl of samples into 300 μl fresh growth medium in intermediate plates (Cat. No. 353077, Falcon® 96-well Clear Round Bottom Microplate, Corning®) to then mix 15 μl of the diluted samples with 15 μl Griess reagent in the wells of a transparent, flat bottom 384-well plate (Cat. No. X7001, Low Profile Microplate, Molecular Devices) that was measured spectrophotometrically at 540 nm (EnVision, Perkin Elmer®).

**2.4.3 Quantification of the nitrification inhibition**

To assess the efficacy of the compounds (in terms of nitrification inhibition) and to enable comparison between plates and batches of cultures, we calculated the relative nitrification. More in detail, all nitrite results were normalized towards both a negative control, containing no compound, and a positive control, containing the benchmark (100 μM DMP), using Equation 1. This normalization was done per multi-well plate and each plate contained 32 positive and 32 negative controls.

$$Relative\ nitrification = \frac{Nitrite(compound) - Nitrite(positive\ \ control)}{Nitrite(negative\ control) - Nitrite(positive\ control)} \quad \text{(Equation 1)}$$

As a result, compounds that allowed full nitrification (no nitrification inhibition) show a relative nitrification of 1 (or 100%). A compound that shows the same nitrification inhibition as the positive control shows a nitrification inhibition of 0.

IC50 values, that is the concentration that inhibits 50% of the nitrification, were calculated after fitting a logistic curve to 8-point dose response data, using doses between 2 and 100 μM with 1.75 fold steps. If a concentration of 2 μM already inhibited nitrification, the IC50 value could not be calculated. In that case, IC50 values represent the dose that gives inhibition closest to 50%.

**Cell growth inhibition assays**

The studied structures have (at least in the tested assays) no toxic/aspecific effects. There is no effect on AOA systems (ABIL, from Avecom, and Nitrososphaera viennensis) and no effect on microtox.

**2.6 Nitrosomonas europaea ammonia vs. hydroxylamine oxidation assays**

To determine if compounds specifically inhibited NH3 or NH2OH oxidation, N. europaea cells were provided with either NH3 or hydroxylamine (NH2OH) as N-source. Two days old, late-log phase cultures with a NO2- concentration between 10 and 20 mM were washed 3 times in fresh growth medium (without N) and finally 4 times overconcentrated via centrifugation (4000 rpm, 15 min, 4°C). Transparent, flat bottom 96-well plate (Cat. No. 353072, Falcon® 96 Well Clear Flat Bottom Microplate, Corning®) were filled with 150 μl culture per well using a dispenser (Multidrop™ Combi Reagent Dispenser, Thermo Scientific™). Compounds (final concentration 50 μM) were added in triplicate to the multi-well plate by use of a Tecan robot (Freedom EVO®, Tecan). Thiourea (positive control for inhibition of NH3 oxidation - final concentration 100 μM) and phenylhydrazine hydrochloride (positive control for inhibition of NH2OH oxidation - final concentration 1 mM) were added to the outer two columns. Each plate was made in duplicate to add either 500 μM (NH4)2SO4 (final [NH4+] = 1 mM) or 1 mM NH2OH. All plates were separately wrapped in Parafilm. Stacks of 4 plates were put on top of a 96-well plate filled with 100 μl MilliQ per well, covered with aluminum foil and shaken (± 150 rpm) at 28°C. To prevent read-out of secondary effects on NO2- production, NO2-was measured only 30 min later. For this, the samples were first 2 times diluted by pipetting 15 μl in 15 μl fresh growth medium in 96-well intermediate plates (Cat. Ref. PCR-96-FS-C, 96-well PCR Microplate, Axygen®) to then mix 15 μl of the diluted samples with 15 μl Griess reagent in the wells of a transparent, flat bottom 384-well plate (Cat. No. X7001, Low Profile Microplate, Molecular Devices) that was measured spectrophotometrically at 540 nm (EnVision, Perkin Elmer®).

**2.7 In soil assays**

Top layer (0 to 10 cm) soil samples were collected from different fields in Belgium (Merelbeke and Moorslede). Vegetation on the field trial was removed and samples were taken from different plots. All soil samples were mixed and sieved (mesh size 2.8 mm) to filter large debris and homogenize soil. The soil was stored at 5°C in plastic containers covered with Saran foil to prevent changes in microbial community composition and to maintain the original soil water content.

Soil water content was determined by drying 20 g soil for ≥ 48 h in a 60°C oven and measuring weight before and after drying. Based on the soil water content (± 20%), compound solutions were prepared so that addition of 200 μl compound and 200 μl NH4Cl solution would result in a final compound concentration of 50 μM and a final $NH_4^+$ concentration of 10 mM. Per treatment, 5 small pots were filled with 20 g soil. Next, the soil was treated first with the respective compound solution and then with the NH4Cl solution. Per tray, positive (50 μM DMP) and negative controls (DMSO) were included. Pots were incubated at 21°C (6 a.m. to 10 p.m. light) for 7 days. Demineralized water was added to the soil every 2 to 3 days to a soil weight of 20 g. In the end, each sample of 20 g soil was dissolved in 100 ml 1M KCl and shaken for 1 hour, followed by filtration through Whatmann® paper. The filtrate was used to measure pH, $NH_4^+$ and NO3-concentrations.

**2.8. Cu-binding assay**

DMSO solutions with compound at a final concentration of 2.5 mM or 10 mM and CuSO4 at a final concentration of 5 mM were compared with pure compound or CuSO4 solutions at the same concentrations. 100 μl of the solution was added to a 96-well plate and the full absorbance spectrum was measured with a SpectraMex2550 plate reader (Molecular devices).

Two agricultural soils coming from fields in Moorslede and Sint-Laureins were used. Screw top vials were filled

with 10 g soil. Vials were sealed with Parafilm and preincubated at 21 °C in the dark for 5 days. After pre-incubation, vials were ventilated using a fan. The 100 μL of a 1 mM compound solution (10 % DMSO) was added, immediately followed by the addition of 100 μL 200 mM NH4Cl. Vials were closed gastight and 5 mL synthetic air was added to the headspace using a plastic syringe, followed by the transfer of 5 mL headspace to an evacuated 3 mL exetainer. Vials were incubated at 21 °C in the dark and background NO concentration was noted. At each sampling timepoint, 5 mL synthetic air was added again to the headspace using a plastic syringe, again followed by the transfer of 5 mL headspace to an evacuated 3 mL exetainer. Vials were ventilated and closed again, and new gas samples were transferred to texetainers like before. N2O, CO2, CH4 in the exetainers were measured via gas chromatography. NO concentration was measured directly.

## 3. Results

The compounds of formula (I) and (II) were tested for their nitrification inhibition effect in two screens, using assays with the nitrifying bacteria N. europaea and N. multiformis. In practice, ammonium and candidate nitrification inhibitors (at 100 μM) were added to a dense bacterial culture in multiwell culture plates and after 24h of incubation, the nitrite level was measured. To normalize for differences between batches of cultures and multiwell culture plates, for each well a relative nitrification was calculated that was normalized towards both the negative and positive controls (see "Material and Methods" above for full details). As such, a relative nitrification of 100% or 0% indicates no nitrification inhibition or nitrification inhibition at the level of the positive control (100 μM 3,4-dimethylpyrazole (DMP)), respectively.

### 3.1 Results in Table 1

The results shown in Table 1 below are based on the use of the nitrifying bacteria Nitrosomonas europaea and Nitrosospira multiformis. The N-heterocyclic compounds of general formula (I) and (II) and (III) and (IV) inhibit the nitrification in at least one the screened systems. The inhibitory activity of this class of molecules was confirmed for a number of structures with varying sub-groups. Eight different doses and four biological repeats were employed. Table 1 shows that all (tested) substances clearly inhibit nitrification in the tested systems, and that some inhibitors show strong inhibition at very low doses (some show still complete inhibition at 2 μM).

As a reference, 3,4-dimethylpyrazole was employed in Comparative Example C1. The IC50 values denote the estimated concentration causing 50% nitrification inhibition. The values were predicted by fitting a logistic curve on 8-point dose response data or based on the lowest tested dose that gives inhibition higher than 50%.

IC50 presents the concentration at which 50% inhibition occurs. Hence, lower values indicate stronger nitrification inhibition. All tested nitrification inhibitors show a stronger effect on Nitrosospira multiformis.

From Table 1 it becomes evident that all (tested) molecules were able to inhibit nitrification. Therefore, the presence of the general formula (I), (II), (III) or (IV) substructure is necessary and seems to be sufficient to achieve the nitrification inhibition. Different side groups on the aminomethyl group in general formula (I) and (III), bound via the nitrogen atom or on the thioether group in general formula (II) and (IV), hardly affect the nitrification inhibition. Addition of molecular structures on the groups R2 and R3 of the structures of general formula (I) and (II) are possible as well, but particularly complex substructures might tend to weaken the nitrification inhibitory capacity. Nevertheless, all molecules of the substructures of general formula (I) and (II) and (III) and (IV) inhibit nitrification to some extent in at least one of the two tested nitrifying bacteria.

Not all molecules shown in Table 1 were tested.

All tested molecules are strong nitrification inhibitors that act specifically on nitrifying bacteria. NA means not analyzed.

**Table 1**

| Example | Structure | *Nitrosomonas europaea* | | *Nitrosospira multiformis* | | |
|---|---|---|---|---|---|---|
| | | Nitrification (%) at tested concentration (0 = maximum activity) | IC50 [μM] | Nitrification (%) at tested concentration (0 = maximum activity) | IC50 [μM] | Tested concentration (μM) |
| C1 | 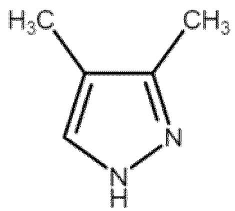 | 0 | 6 | 0 | 14 | 100 |
| 1 | 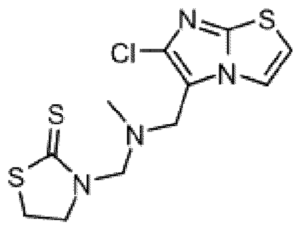 | 0 | <2 | 0 | <0.8 | 100 |
| 2 | 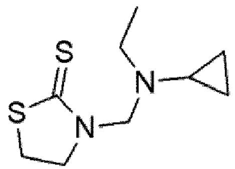 | 0 | <2 | 0 | <2 | 100 |
| 3 | 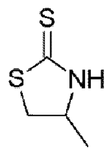 | 0 | <3.5 | 0 | <2 | 100 |
| 4 | 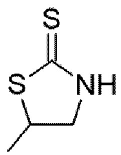 | 0 | <2 | 0 | <2 | 100 |

(continued)

| Example | Structure | *Nitrosomonas europaea* | | *Nitrosospira multiformis* | | |
|---|---|---|---|---|---|---|
| | | Nitrification (%) at tested concentration (0 = maximum activity) | IC50 [μM] | Nitrification (%) at tested concentration (0 = maximum activity) | IC50 [μM] | Tested concentration (μM) |
| 5 | 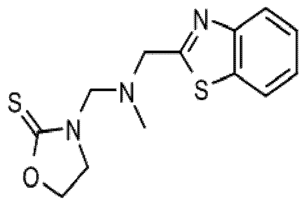 | 0 | 11 | 0 | <3.5 | 100 |
| 6 | 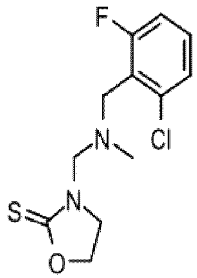 | 0 | 14 | 0 | <3.5 | 100 |
| 7 | 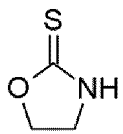 | 0 | <6 | 0 | <10 | 100 |
| 8 | 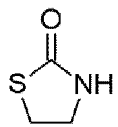 | 47 | <100 | 31 | <100 | 100 |

(continued)

| Example | Structure | *Nitrosomonas europaea* | | *Nitrosospira multiformis* | | |
|---|---|---|---|---|---|---|
| | | Nitrification (%) at tested concentration (0 = maximum activity) | IC50 [μM] | Nitrification (%) at tested concentration (0 = maximum activity) | IC50 [μM] | Tested concentration (μM) |
| 9 | 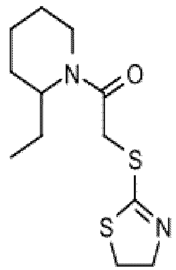 | 68 | >100 | 0 | <6 | 100 |
| 10 | 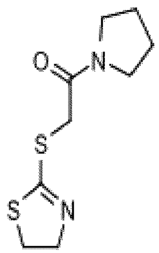 | 81 | >100 | 6 | <10 | 100 |
| 11 | 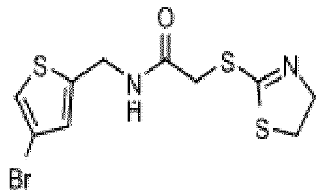 | NA | NA | 0 | <20 | 100 |

(continued)

| Example | Structure | *Nitrosomonas europaea* | | *Nitrosospira multiformis* | | |
|---|---|---|---|---|---|---|
| | | Nitrification (%) at tested concentration (0 = maximum activity) | IC50 [µM] | Nitrification (%) at tested concentration (0 = maximum activity) | IC50 [µM] | Tested concentration (µM) |
| 12 | 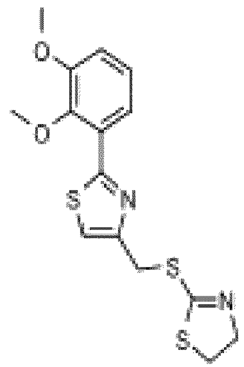 | 0 | <100 | 11 | <20 | 100 |
| 13 | 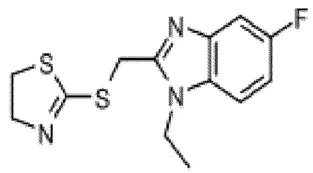 | 73 | NA | 0 | NA | 50 |
| 14 | 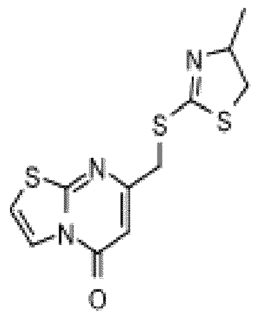 | 0 | <57 | 0 | <20 | 100 |

(continued)

| Example | Structure | *Nitrosomonas europaea* | | *Nitrosospira multiformis* | | |
|---|---|---|---|---|---|---|
| | | Nitrification (%) at tested concentration (0 = maximum activity) | IC50 [μM] | Nitrification (%) at tested concentration (0 = maximum activity) | IC50 [μM] | Tested concentration (μM) |
| 15 | 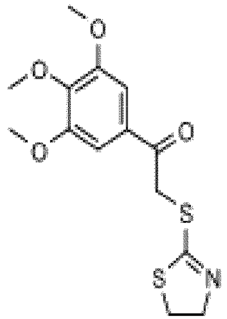 | 78 | NA | 13 | NA | 50 |
| 16 | 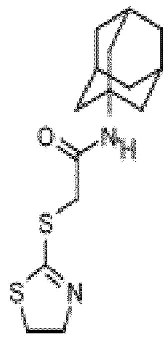 | | | | | |
| 17 | 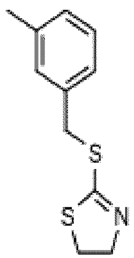 | 100 | NA | 36 | NA | 50 |

(continued)

| Example | Structure | *Nitrosomonas europaea* | | *Nitrosospira multiformis* | | |
|---|---|---|---|---|---|---|
| | | Nitrification (%) at tested concentration (0 = maximum activity) | IC50 [μM] | Nitrification (%) at tested concentration (0 = maximum activity) | IC50 [μM] | Tested concentration (μM) |
| 18 | 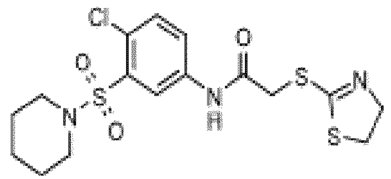 | | | | | |
| 19 | 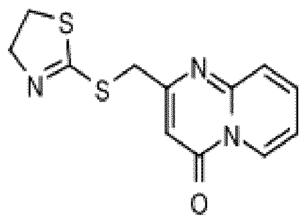 | | | | | |
| 20 | 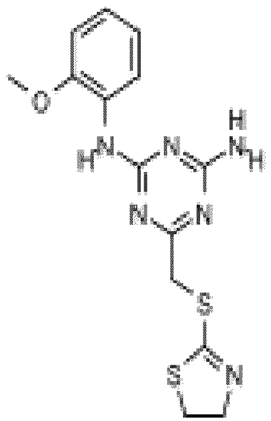 | 79 | NA | 42 | NA | 50 |

(continued)

| Example | Structure | *Nitrosomonas europaea* | | *Nitrosospira multiformis* | | |
|---|---|---|---|---|---|---|
| | | Nitrification (%) at tested concentration (0 = maximum activity) | IC50 [μM] | Nitrification (%) at tested concentration (0 = maximum activity) | IC50 [μM] | Tested concentration (μM) |
| 21 | 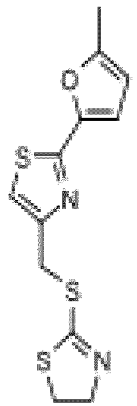 | | | | | |
| 22 | 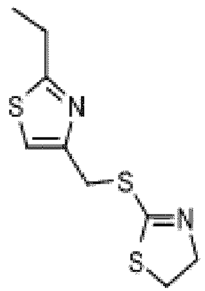 | | | | | |

(continued)

| Example | Structure | *Nitrosomonas europaea* | | *Nitrosospira multiformis* | | |
|---|---|---|---|---|---|---|
| | | Nitrification (%) at tested concentration (0 = maximum activity) | IC50 [μM] | Nitrification (%) at tested concentration (0 = maximum activity) | IC50 [μM] | Tested concentration (μM) |
| 23 | 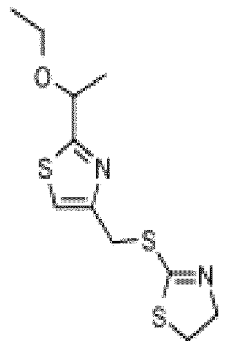 | | | | | |
| 24 | 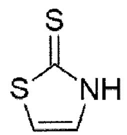 | 1 | 13 | 0 | 1.3 | 100 |
| 25 | 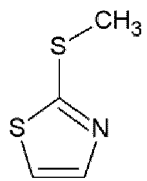 | 2 | 12 | 53 | 140 | 100 |
| 26 | 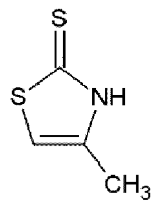 | 0 | 38 | 0 | 2.1 | 100 |
| 27 | 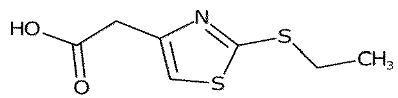 | 62 | >100 | 77 | >100 | 100 |

(continued)

| Example | Structure | *Nitrosomonas europaea* | | *Nitrosospira multiformis* | | |
|---|---|---|---|---|---|---|
| | | Nitrification (%) at tested concentration (0 = maximum activity) | IC50 [μM] | Nitrification (%) at tested concentration (0 = maximum activity) | IC50 [μM] | Tested concentration (μM) |
| 28 | 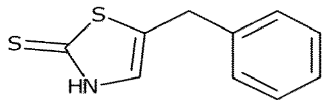 | 65 | >100 | 0 | 12 | 100 |
| 29 | 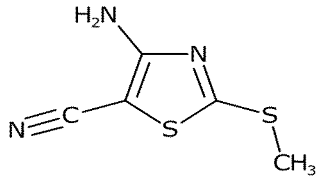 | 0 | 28 | 61 | >100 | 100 |
| 30 | 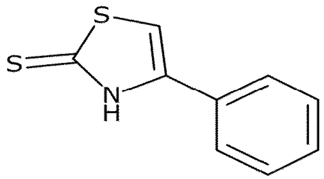 | 21 | 42 | 0 | 36 | 100 |
| 31 | 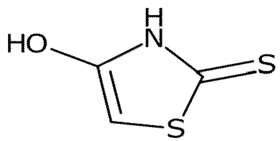 | 12 | 14 | 11 | 16 | 100 |
| 32 | 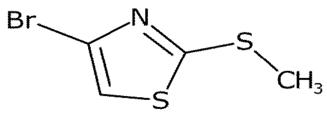 | 46 | <100 | 75 | >100 | 100 |
| 33 | 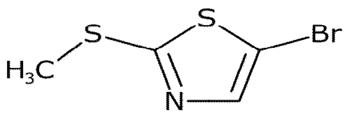 | 68 | >100 | 64 | >100 | 100 |

(continued)

| Example | Structure | *Nitrosomonas europaea* | | *Nitrosospira multiformis* | | |
|---|---|---|---|---|---|---|
| | | Nitrification (%) at tested concentration (0 = maximum activity) | IC50 [μM] | Nitrification (%) at tested concentration (0 = maximum activity) | IC50 [μM] | Tested concentration (μM) |
| 34 | 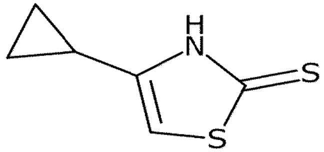 | 0 | 52 | 0 | 11 | 100 |
| 35 | 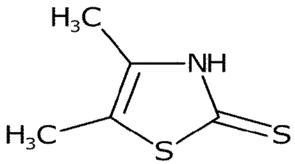 | 12 | 43 | 0 | 3 | 100 |
| 36 | 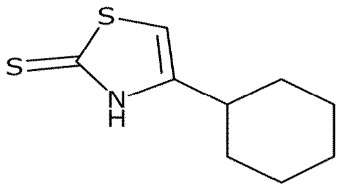 | 0 | 16 | 15 | 49 | 100 |
| 37 | 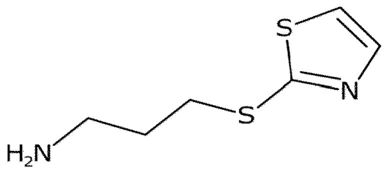 | 58 | >100 | 7 | 44 | 100 |
| 38 | 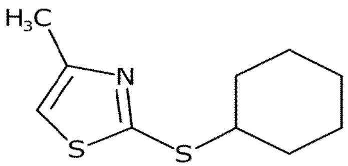 | 92 | >100 | 53 | >100 | 100 |

(continued)

| Example | Structure | *Nitrosomonas europaea* | | *Nitrosospira multiformis* | | |
|---|---|---|---|---|---|---|
| | | Nitrification (%) at tested concentration (0 = maximum activity) | IC50 [μM] | Nitrification (%) at tested concentration (0 = maximum activity) | IC50 [μM] | Tested concentration (μM) |
| 39 | 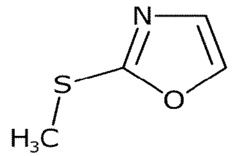 | 22 | 26 | 52 | >100 | 100 |
| 40 | 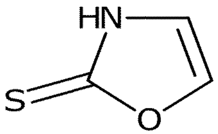 | 0 | 4 | 0 | 3 | 100 |
| 41 | 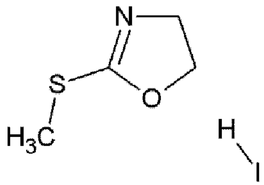 | 88 | >100 | 68 | >100 | 100 |
| 42 | 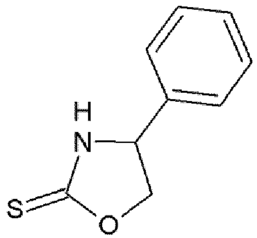 | 40 | 68 | 12 | <57 | 100 |
| 43 | 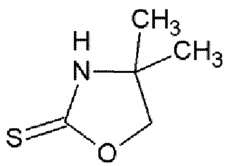 | 0 | 7 | 0 | 20 | 100 |

(continued)

| Example | Structure | *Nitrosomonas europaea* | | *Nitrosospira multiformis* | | |
|---|---|---|---|---|---|---|
| | | Nitrification (%) at tested concentration (0 = maximum activity) | IC50 [μM] | Nitrification (%) at tested concentration (0 = maximum activity) | IC50 [μM] | Tested concentration (μM) |
| 44 | 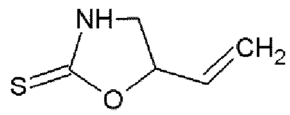 | 0 | 2 | 0 | 24 | 100 |

Particularly, the molecule of Example 1, 3-[[(6-chloroimidazo[2,1-b][1,3]thiazol-5-yl)methyl-methylamino] methyl]-1,3-thiazolidine-2-thione strongly inhibited nitrification on both tested bacteria. Two similar molecules, 3-[[1,3-benzothiazol-2-ylmethyl(methyl)amino]methyl]-1,3-oxazolidine-2-thione and 3-[[(2-chloro-6-fluorophenyl)methyl-methylamino]methyl]-1,3-oxazolidine-2-thione of Examples 5 and 6, respectively, showed a strong nitrification inhibition towards the two ammonia oxidizing bacteria.

These three structures of Examples 1, 5 and 6 that are particularly preferred are shown below:

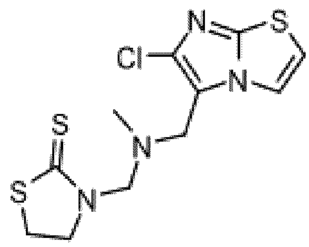
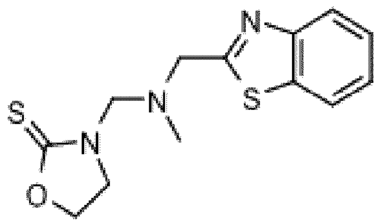
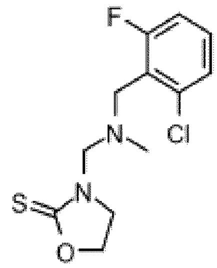

3.2 **Ammonia** vs hydroxylamine **oxidation** assay

To get insights on the targeted metabolic pathway and to discriminate between compounds that specifically affect NH3 oxidation or that affect another pathway, three representative novel nitrification inhibitors (Examples 1, 5 and 6) were tested in the "ammonia vs hydroxylamine oxidation assay" in which NO2- production from NH3 was compared with NO2- production from NH2OH. As NH3 is converted into NH2OH before NO2- is produced, comparing nitrification inhibition of the new compounds towards NH3 (see "1." in the scheme below) versus NH2OH (see "2." in the scheme below) indicates which part of the pathway is inhibited.

No nitrification inhibitor:

$$NH_3 \xrightarrow{ammonia\ oxidation} NH_2OH \xrightarrow{hydroxylamine\ oxidation} NO_2^-$$

$$NH_2OH \rightarrow NO_{2-}$$

Non-specific or general metabolic inhibitor:

$$NH_3 \rightarrow X$$

$$NH_2OH \rightarrow X$$

Ammonia oxidation-specific inhibitor:

$$NH_3 \rightarrow X$$

$$NH_2OH \rightarrow NO_{2-}$$

Ammonia oxidation is an essential step for the metabolism of nitrifying bacteria. Therefore, inhibition of ammonia oxidation by a nitrification inhibitor will indirectly affect all other enzymatic steps and will affect the second step as well, albeit less strong than the first step. Indeed, even in a 30 minutes assay, DMP, known to target ammonia oxidation, also reduced NH2OH oxidation, but in a lesser extent than the effect on ammonia oxidation.

The below Table 2 shows that all three new nitrification inhibitors clearly inhibit ammonia oxidation, but hardly hydroxylamine oxidation. The limited effect on hydroxylamine oxidation is most likely an indirect effect as inhibiting ammonia oxidation will affect the whole metabolism. Indeed, DMP, known to target the first step, has also a limited effect on the second step in the assay (Table 2). Hence, the new nitrification inhibitors specifically target ammonia oxidation (the first step of nitrification), which further corroborates that they specifically act on AOBs and are not generally toxic.

**Table 2**

| Example | Structure | Nitrification from NH3 (% compared to DMSO) | Nitrification from NH2OH (% compared to DMSO) |
|---|---|---|---|
| C1 | 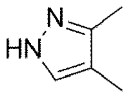 | 27 | 56 |
| 1 | 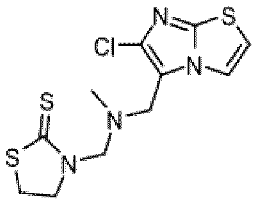 | 12 | 81 |
| 5 | 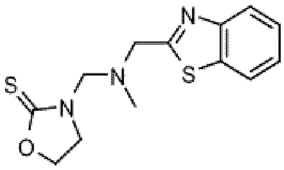 | 17 | 77 |
| 6 | 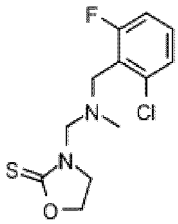 | 18 | 80 |

### 3.3 Test performed in agricultural soils

Finally, the effect of a representative set of the new nitrification inhibitors was tested in soil.

The nitrification inhibitors were applied in a nitrification inhibition assay using agricultural soil. In practice, ammonium with or without the new nitrification inhibitors was added to soil from a field in Merelbeke (Belgium). The ammonium level was measured at the start of the experiment and at the end of the experiment, after one week of incubation. A nitrification inhibition percentage was calculated by comparing the reduction in ammonium level with the nitrification inhibitor to the reduction in ammonium without the nitrification inhibitor.

Ammonium was added to soil with a high nitrification activity and treated with the nitrification inhibitors (final concentration of 50 μM). Table 3 shows that, after one week of treatment, especially the thiazolidine-containing compounds of Example 1 strongly inhibited the ammonium consumption. Also the thiazolidine-containing compounds that form thio-ethers (Examples 9 and 10) and the oxazolidine-containing compound of Example 5 showed significant inhibition. This validates that the new nitrification inhibitors are effectively inhibiting ammonia oxidation by soil communities.

**Table 3**

| Example | Structure | % Nitrification inhibition in soil (7 days of incubation, Merelbeke soil) |
|---|---|---|
| 1 | 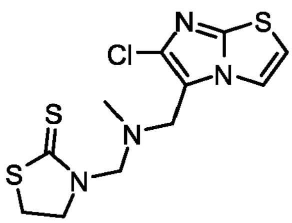 | 60 |

(continued)

| Example | Structure | % Nitrification inhibition in soil (7 days of incubation, Merelbeke soil) |
|---|---|---|
| 5 | 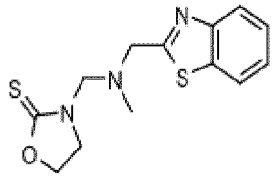 | 14 |
| 9 | 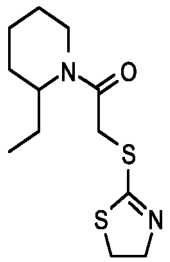 | 10 |
| 10 | 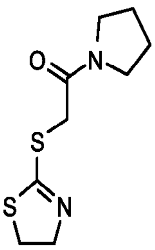 | 7 |

It was found that presence of a thiazolidine-thiol substructure can be associated to nitrification inhibition capacity. Evaluation of different structural variants show that thiazolidine-thiol- and oxazolidine-thiol-containing structures are preferred nitrification inhibitors. Preferred compounds have a substructure that is bound via an amino-methyl bound to a more complex structure. Such molecules can also form the thiol tautomers if R1 is hydrogen in general formula (I). Preferred are the compounds containing a thiazolidine-thiol substructure, an oxazolidine-thiol substructure, or a thiazolidine-thioether substructure.

A set of examples was tested in the same conditions (Moorslede soil, 3 days of incubation) at a concentration of 50 μM. The results are shown in Table 4.

**Table 4**

| Example | Structure | % Nitrification inhibition in soil (3 days of incubation, Moorslede soil) at 50 μM |
|---|---|---|
| 7 | 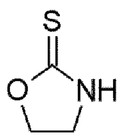 | 100 |
| 24 | 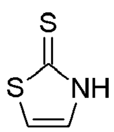 | 40 |
| 25 | 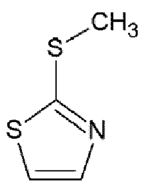 | 73 |

Another set of examples was tested in different concentrations in Moorslede soil as well, and were sampled after

5 days. The results are shown in Table 5. Table 3-7 show that diverse variations within the claimed general formula effectively inhibit nitrification in soil.

**Table 5**

| Example | Structure | % Nitrification inhibition in soil (5 days of incubation, Moorslede soil) at indicated concentration | | |
|---|---|---|---|---|
| | | 50 μM | 20 μM | 5 μM |
| 7 | 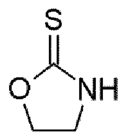 | 38 | 32 | 8 |
| 25 | 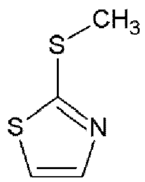 | 30 | 14 | 3 |
| 39 | 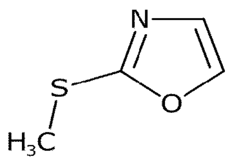 | 62 | NA | NA |

**3.4 Nitrification inhibition assay employing 3H-1,3-thiazole-2-thione**

The compound of Example 24, 3H-1,3-thiazole-2-thione was employed in a full dose-response assay in the two ammonia-oxidizing bacteria Nitrosomonas europaea and Nitrosospira multiformis in accordance with above sections 1., 2.4.1 to 2.4.3.

**Table 6**

| Tested concentration [μM] | | 2 | 3.5 | 6 | 11 | 19 | 33 | 57 | 100 |
|---|---|---|---|---|---|---|---|---|---|
| Average nitrification [%] in Nitrosomonas europaea | 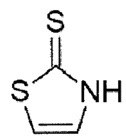 | 79 | 78 | 76 | 74 | 60 | 43 | 20 | 1 |
| | 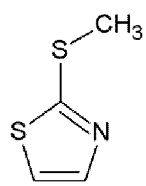 | 85 | 76 | 73 | 58 | 50 | 32 | 20 | 2 |
| | 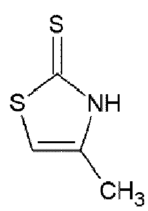 | 91 | 84 | 80 | 83 | 78 | 71 | 50 | 0 |

(continued)

| Tested concentration [μM] | | 2 | 3.5 | 6 | 11 | 19 | 33 | 57 | 100 |
|---|---|---|---|---|---|---|---|---|---|
| Average nitrification [%] in Nitrosospira multiformis | 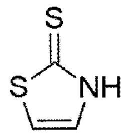 | 25 | 4 | 0 | 0 | 0 | 0 | 0 | 0 |
| | 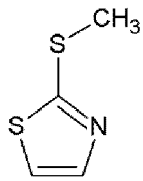 | 81 | 80 | 80 | 76 | 74 | 67 | 62 | 53 |
| | 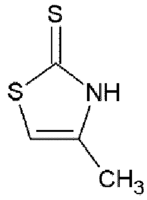 | 51 | 27 | 6 | 2 | 0 | 0 | 0 | 0 |

Concentrations ranging from 2 to 100 μM of 3H-1,3-thiazole-2thione (tautomeric form being 3H-1,3-thiazole-2-thiol), 2-(methylsulfanyl)-1,3-thiazole and 4-methyl-1,3-thiazole-2-thiol were tested. The results are shown in the following Table 5. The results for 100 μM are also contained in Table 1.

3H-1,3-thiazole-2-thione has a very strong effect towards Nitrosospira multiformis (only 25% nitrification after application of 2 μM), but also still nearly completely inhibits nitrification in Nitrosomonas europaea using higher concentrations.

### 3.5 Test performed in agricultural soil

The effect of 3H-1,3-thiazole-2-thione was tested in soil in accordance with the test described above in section 3.3. The results are summarized in the following Table 7.

**Table 7**

| Name | Structure | Average % nitrification inhibition in soil (5 days of incubation, Moorslede soil) at 20 μM |
|---|---|---|
| 3H-1,3-thiazole-2-thione | 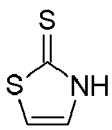 | 014 |
| 1,3-thiazolidine-2-thione | 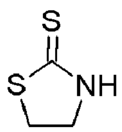 | 7 |

3H-1,3-thiazole-2-thione effectively inhibits nitrification in soil.

In the presented data, the extent of inhibition seems to be limited, but this experiment was intentionally conducted at a low concentration (20 μM) to allow comparison of efficacy between different inhibitors.

### 3. 6 Inhibition of greenhouse gas emissions

To further confirm the inhibitory effect of the new class of nitrification inhibitors on nitrogen losses, gas emissions from soil fertilized with ammonium and treated with 1,3-thiazolidine-2-thione were captured. NO, N2O, CO2 and CH4 was measured. Table 7 shows the cumulative values during an incubation of 99h, and shows that especially NO and N2O, but

also $CO_2$, are strongly reduced upon use of the new nitrification inhibitor, which was the case in two different agricultural soils. No significant effects could be detected on $CH_4$.

**Table 8**

| | | | Cumulative emission at indicated timepoint [ppm] | | | | |
|---|---|---|---|---|---|---|---|
| Soil | Greenhouse gas | Treatment | 4h | 24h | 48h | 72h | 99h |
| Moor slede | N2O | DMSO (no inhibitor) | 226 | 1895 | 3733 | 4466 | 4466 |
| | | 1,3-thiazolidine-2-thione | 0 | 0 | 0 | 0 | 0 |
| | NO | DMSO (no inhibitor) | 263 | 614 | 1014 | 1162 | 1160 |
| | | 1,3-thiazolidine-2-thione | 25 | 28 | 43 | 65 | 65 |
| | CO2 | DMSO (no inhibitor) | 724 | 2825 | 5175 | 7359 | 9431 |
| | | 1,3-thiazolidine-2-thione | 619 | 2385 | 4261 | 6195 | 8131 |
| | CH4 | DMSO (no inhibitor) | 0.06 | 0.29 | 0.29 | 0.32 | 0.34 |
| | | 1,3-thiazolidine-2-thione | 0.20 | 0.38 | 0.38 | 0.51 | 0.51 |
| Sint-Laureins | N2O | DMSO (no inhibitor) | 42 | 481 | 1065 | 1587 | 1730 |
| | | 1,3-thiazolidine-2-thione | 0 | 0 | 1 | 1 | 2 |
| | NO | DMSO (no inhibitor) | 92 | 228 | 423 | 631 | 713 |
| | | 1,3-thiazolidine-2-thione | 17 | 52 | 86 | 140 | 161 |
| | CO2 | DMSO (no inhibitor) | 382 | 1367 | 3041 | 5330 | 7596 |
| | | 1,3-thiazolidine-2-thione | 372 | 1179 | 2094 | 3101 | 4168 |
| | CH4 | DMSO (no inhibitor) | 0.18 | 0.22 | 0.25 | 0.35 | 0.35 |
| | | 1,3-thiazolidine-2-thione | 0.00 | 0.10 | 0.16 | 0.25 | 0.25 |

**4. New nitrification inhibitors are Cu-chelators**

The new nitrification inhibitors form complexes with Cu.

As multiple known nitrification inhibitors are Cu-chelators, the Cu-chelating ability was tested by analyzing the absorbance spectra with or without Cu for the new inhibitors. If Cu forms a complex with the nitrification inhibitor, the absorbance spectra are expected to be shifted. Indeed, the absorbance spectra of the new inhibitors clearly shifted if combined with Cu, indicating that they form complexes with Cu, and further corroborating their action as a nitrification inhibitor.

## Claims

1. The use of an N-heterocyclic compound of the general formula (a)

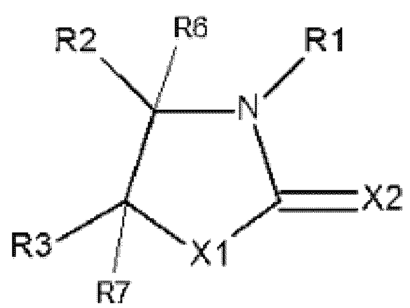

(a)

with the following definitions:

X1 being S, X2 being O and at least one of X1 and X2 being S
R2 H or C1-4-alkyl,
R3 H or C1-4-alkyl
R6 and R7 are hydrogen or together form a covalent carbon-carbon bond in general formula (a) R1 being H, C1-12-alkyl or -CH2-NR4R5 with
R4 hydrogen or C1-4-alkyl,
R5 C1-12-hydrocarbon residue which can contain one to three halogen atoms and/or one to four heteroatoms, selected from the group consisting of nitrogen, oxygen and sulfur, it also being possible for R4 and R5, together with the nitrogen atom joining them, to form a 5- or 6-membered saturated or unsaturated heterocyclic radical, which optionally may also contain one or two further heteroatoms selected from the group consisting of nitrogen, oxygen and sulfur,
as nitrification inhibitor,
wherein the N-heterocyclic compound of the general formula (a) is an N-heterocyclic compound of the general formula (III)

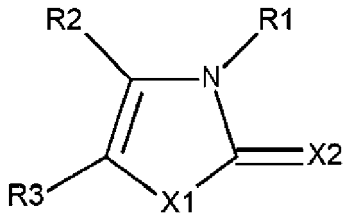

(III)

with the following definitions:
X1 being S, X2 being O and at least one of X1 and X2 being S
R2 H or C1-4-alkyl,
R3 H or C1-4-alkyl
in general formula (III) R1 being H, C1-12-alkyl or -CH2-NR4R5 with R4 hydrogen or C1-4-alkyl,
R5 C1-12-hydrocarbon residue which can contain one to three halogen atoms and/or one to four heteroatoms, selected from the group consisting of nitrogen, oxygen and sulfur, it also being possible for R4 and R5, together with the nitrogen atom joining them, to form a 5- or 6-membered saturated or unsaturated heterocyclic radical, which optionally may also contain one or two further heteroatoms selected from the group consisting of nitrogen, oxygen and sulfur.

2. The N-heterocyclic compound for use as defined in any one of the preceding claims, wherein in general formula (III) R5 is a C3-10-hydrocarbon residue which can contain one to two halogen atoms and/or one to three heteroatoms and which contains at least one cyclic structure, wherein preferably in general formula (III) R5 is a C3-8-hydrocarbon residue which can contain one to two halogen atoms and/or one to three heteroatoms and which contains a 5- or 6-membered cyclic structure which can be annellated to a second 5- or 6-membered cyclic structure, wherein more preferably in formula (III) R1, R2 and R3 are independently hydrogen, methyl or ethyl.

3. The use as claimed in one of claims 1 to 2, wherein the N-heterocyclic compound is an N-heterocyclic compound of the following formula:

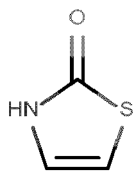

4. The use of an N-heterocyclic compound as defined in any one of the preceding claims for use as an additive or coating material for inorganic and/or organic and/or organomineral fertilizers, preferably inorganic fertilizers, more preferably ammonium- and/or urea-containing nitrogen fertilizers.

5. The use as claimed in any one of the preceding claims, wherein the N-heterocyclic compound is delivered in a form of a formulation, solution or dispersion, separately or simultaneously with a fertilizer, or is incorporated into the fertilizer or

is applied to the fertilizer.

6. The use of an N-heterocyclic compound as defined in one of the preceding claims for reducing the nitrogen or carbon losses in inorganic and/or organic and/or organomineral fertilizers or nitrogen- or carbon-containing compounds or materials and also on harvest refuse and on grazed land or during the storage of liquid manure and for lowering the ammonia load in animal stalls.

7. The use as claimed in one of the preceding claims, wherein the N-heterocyclic compound is used together with at least one additional agrochemical agent, preferably selected from the group consisting of

at least one further nitrification inhibitor, preferably selected from the group consisting of 2-(3,4-dimethyl-pyrazol-1-yl)-succinic acid (DMPSA), 3,4-dimethylpyrazole (DMP), 3,4-dimethylpyrazolephosphate (DMPP), dicyandiamide (DCD), 1H-1,2,4-triazole, 3-methylpyrazole (3-MP), 2-chloro-6-(trichloromethyl)-pyridine, 5-ethoxy-3-trichloromethyl-1,2,4-thiadiazol, 2-amino-4-chloro-6-methyl-pyrimidine, 2-mercapto-benzothiazole, 2-sulfanilamidothiazole, thiourea, sodium azide, potassium azide, 1-hydroxypyrazole, 2-methylpyrazole-1-carboxamide, 4-amino-1,2,4-triazole, 3-mercapto-1,2,4-triazole, 2,4-diamino-6-trichloromethyl-5-triazine, carbon bisulfide, ammonium thiosulfate, sodium trithiocarbonate, 2,3-dihydro-2,2-dimethyl-7-benzofuranol methyl carbamate and N-(2,6-dimethylphenyl)-N-(methoxyacetyl)-alanine methyl ester,
at least one urease inhibitor, preferably selected from N-n-butylthiophosphoric triamide (NBTPT) and/or N-n-propylthiophosphoric triamide (NPTPT),
at least one customary agrochemical auxiliary agent, preferably selected from the group consisting of aqueous and/or organic solvents, pH-adjusting agents, surfactants, wetting agents, spreading agents, adhesion promoters, carriers, fillers, viscosity-adjusting agents, emulsifiers, dispersants, sequestering agents, anti-settling agents, coalescing agents, rheology modifiers, defoaming agents, photo-protectors, anti-freeze agents, biostimulants, pesticides, biocides, plant growth regulators, safeners, penetrants, anticaking agents, mineral and/or vegetable oils and/or waxes, colorants and drift control agents,
and mixtures thereof.

8. A mixture, containing at least one N-heterocyclic compound as defined in one of the preceding claims,

and at least one additional agrochemical agent, preferably selected from the group consisting of
at least one further nitrification inhibitor, preferably selected from the group consisting of 2-(3,4-dimethyl-pyrazol-1-yl)-succinic acid (DMPSA), 3,4-dimethylpyrazole (DMP), 3,4-dimethylpyrazolephosphate (DMPP), dicyandiamide (DCD), 1H-1,2,4-triazole, 3-methylpyrazole (3-MP), 2-chloro-6-(trichloromethyl)-pyridine, 5-ethoxy-3-trichloromethyl-1,2,4-thiadiazol, 2-amino-4-chloro-6-methyl-pyrimidine, 2-mercapto-benzothiazole, 2-sulfanilamidothiazole, thiourea, sodium azide, potassium azide, 1-hydroxypyrazole, 2-methylpyrazole-1-carboxamide, 4-amino-1,2,4-triazole, 3-mercapto-1,2,4-triazole, 2,4-diamino-6-trichloromethyl-5-triazine, carbon bisulfide, ammonium thiosulfate, sodium trithiocarbonate, 2,3-dihydro-2,2-dimethyl-7-benzofuranol methyl carbamate and N-(2,6-dimethylphenyl)-N-(methoxyacetyl)-alanine methyl ester,
at least one urease inhibitor, preferably selected from N-n-butylthiophosphoric triamide (NBTPT) and/or N-n-propylthiophosphoric triamide (NPTPT),
at least one customary agrochemical auxiliary agent, preferably selected from the group consisting of aqueous and/or organic solvents, pH-adjusting agents, surfactants, wetting agents, spreading agents, adhesion promoters, carriers, fillers, viscosity-adjusting agents, emulsifiers, dispersants, sequestering agents, anti-settling agents, coalescing agents, rheology modifiers, defoaming agents, photo-protectors, anti-freeze agents, biostimulants, pesticides, biocides, plant growth regulators, safeners, penetrants, anticaking agents, mineral and/or vegetable oils and/or waxes, colorants and drift control agents,
and mixtures thereof.

9. A fertilizer mixture, containing

A. an inorganic and/or organic and/or organomineral fertilizer and
B. 10 to 10000 weight-ppm, based on the inorganic fertilizer, of at least one N-heterocyclic compound as defined in one of the preceding claims.

10. The fertilizer mixture as claimed in claim 9, wherein the fertilizer mixture is in solid form and the N-heterocyclic compound of (III) is applied to the surface of the, preferably inorganic, fertilizer.

**11.** The fertilizer mixture as claimed in one of claims 9 or 10, wherein the fertilizer mixture contains at least one additional agrochemical agent, preferably selected from the group consisting of

at least one further nitrification inhibitor, preferably selected from the group consisting of 2-(3,4-dimethylpyrazol-1-yl)-succinic acid (DMPSA), 3,4-dimethylpyrazole (DMP), 3,4-dimethylpyrazolephosphate (DMPP), dicyandiamide (DCD), 1H-1,2,4-triazole, 3-methylpyrazole (3-MP), 2-chloro-6-(trichloromethyl)-pyridine, 5-ethoxy-3-trichloromethyl-1,2,4-thiadiazol, 2-amino-4-chloro-6-methyl-pyrimidine, 2-mercapto-benzothiazole, 2-sulfanilamidothiazole, thiourea, sodium azide, potassium azide, 1-hydroxypyrazole, 2-methylpyrazole-1-carboxamide, 4-amino-1,2,4-triazole, 3-mercapto-1,2,4-triazole, 2,4-diamino-6-trichloromethyl-5-triazine, carbon bisulfide, ammonium thiosulfate, sodium trithiocarbonate, 2,3-dihydro-2,2-dimethyl-7-benzofuranol methyl carbamate and N-(2,6-dimethylphenyl)-N-(methoxyacetyl)-alanine methyl ester,
at least one urease inhibitor, preferably selected from N-n-butylthiophosphoric triamide (NBTPT) and/or N-n-propylthiophosphoric triamide (NPTPT),
at least one customary agrochemical auxiliary agent, preferably selected from the group consisting of aqueous and/or organic solvents, pH-adjusting agents, surfactants, wetting agents, spreading agents, adhesion promoters, carriers, fillers, viscosity-adjusting agents, emulsifiers, dispersants, sequestering agents, anti-settling agents, coalescing agents, rheology modifiers, defoaming agents, photo-protectors, anti-freeze agents, biostimulants, pesticides, biocides, plant growth regulators, safeners, penetrants, anticaking agents, mineral and/or vegetable oils and/or waxes, colorants and drift control agents,
and mixtures thereof.

**12.** A process for producing the fertilizer mixture as claimed in one of claims 8 to 11 by introducing the N-heterocyclic compound into the fertilizer, and/or applying the N-heterocyclic compound to the surface of the fertilizer.

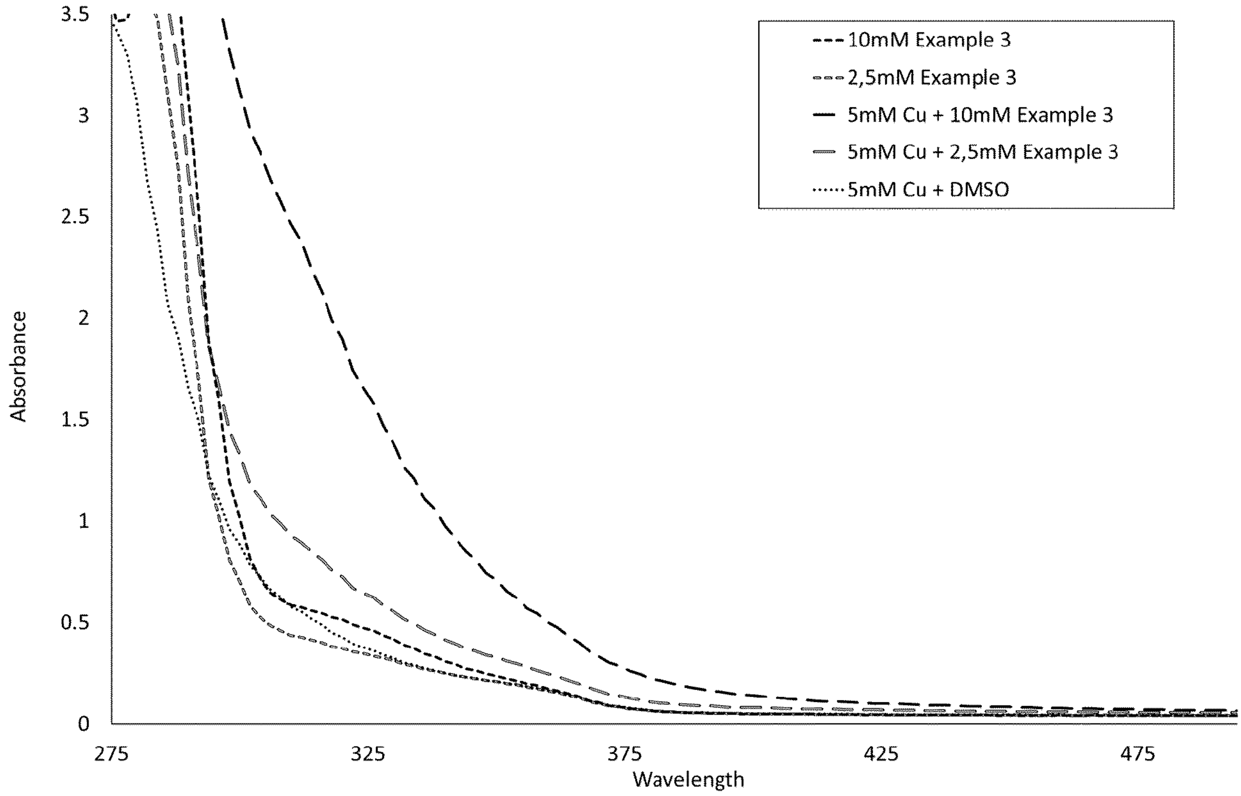
10mM Example 3
2,5mM Example 3
5mM Cu + 10mM Example 3
5mM Cu + 2,5mM Example 3
5mM Cu + DMSO
Absorbance
Wavelength
3.5
3
2.5
2
1.5
1
0.5
0
275
325
375
425
475

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1120388 B
- WO 9624566 A
- WO 2011032904 A
- WO 2013121384 A
- WO 2020020765 A1
- WO 2020020777 A1
- CN 103524159 A
- WO 2020020765 A
- WO 2020020777 A
- WO 2016207210 A
- DE 4128828 A
- DE 10230593 C
- EP 1820788 A
- US 6139596 A
- WO 2015086823 A

### Non-patent literature cited in the description


- **SAHA et al.** *J. Heterocyclic. Chem.*, 2010, vol. 47, 838